# EUROPÄISCHE PATENTSCHRIFT

(11) **EP 1 405 666 B1**
(45) Veröffentlichungstag und Bekanntmachung des Hinweises auf die Patenterteilung: **21.03.2007**
(21) Anmeldenummer: 03028140.6
(22) Anmeldetag: 27.08.1999
(51) Int. Cl.: B01J 19/00, G06F 19/00, B01L 3/00

(54) **Träger für Analytbestimmungsverfahren und Verfahren zur Herstellung des Trägers**
Substrate for methods to determine analytes and methods to manufacture such substrates
Substrats pour procédés de détermination d'analytes et méthodes de fabrication de tels substrats

(30) Priorität: 28.08.1998 DE 19839256; 28.08.1998 DE 19839254; 28.08.1998 DE 19839255; 19.02.1999 DE 19907080; 27.05.1999 DE 19924327
(43) Veröffentlichungstag der Anmeldung: 07.04.2004
(62) Teilanmeldung aus: 99944538.0
(73) Patentinhaber: febit biotech GmbH, 69120 Heidelberg (DE)
(72) Erfinder: Cord F. Stähler, 69469 Weinheim (DE); Peer F. Stähler, 68167 Mannheim (DE); Manfred Müller, 80689 München (DE); Fritz Stähler, 69469 Weinheim (DE); Hans Lindner, 70569 Stuttgart (DE)
(74) Vertreter: Weiss, Wolfgang

(56) Entgegenhaltungen:
- EP-A- 0 721 016
- US-A- 5 571 639
- RAYNER S ET AL: "MerMade: An oligodeoxyribonucleotide synthesizer for high throughput oligonucleotide production in dual 96-well plates" PCR METHODS AND APPLICATIONS, COLD SPRING HARBOR, NY, US, Bd. 8, Nr. 7, 1. Juli 1998 (1998-07-01), Seiten 741-747, XP002089330 ISSN: 1054-9803

## Beschreibung

### 1 Anwendungsgebiet der Erfindung

### 1.1 Hintergrund

Für die Grundlagenforschung in den Biowissenschaften und für die medizinische Diagnostik sowie einige andere Disziplinen ist die präzise Detektion biologisch relevanter Moleküle in definiertem Untersuchungsmaterial von herausragender Bedeutung. Dabei liegt die genetische Information in Form einer enormen Vielfalt von unterschiedlichen Nukleinsäuresequenzen vor, der DNA. Die Realisation dieser Information führt über die Herstellung von Abschriften der DNA in RNA meist zur Synthese von Proteinen, die ihrerseits häufig an biochemischen Reaktionen beteiligt sind.

Die Detektion von bestimmten Nukleinsäuren und die Bestimmung der Abfolge der vier Basen in der Kette der Nukleotide, die generell als Sequenzierung bezeichnet wird, liefert wertvolle Daten für Forschung und angewandte Medizin. In der Medizin konnte in stark zunehmendem Masse durch die in vitro-Diagnostik (IVD) ein Instrumentarium zur Bestimmung wichtiger Patientenparameter entwickelt und dem behandelnden Arzt zur Verfügung gestellt werden. Für viele Erkrankungen wäre eine Diagnose zu einem ausreichend frühen Zeitpunkt ohne dieses Instrumentarium nicht möglich. Hier hat sich die genetische Analyse als wichtiges neues Verfahren etabliert, z.B. für Infektionskrankheiten wie HIV und HBV, genetische Prädisposition für bestimmte Krebsarten oder andere Erkrankungen, Forensik und eine Vielzahl weiterer Anwendungsgebiete. In enger Verzahnung von Grundlagenforschung und klinischer Forschung konnten die molekularen Ursachen und (pathologischen) Zusammenhänge einiger Krankheitsbilder bis auf die Ebene der genetischen Information zurückverfolgt und aufgeklärt werden. Diese Entwicklung steht allerdings noch am Anfang, und gerade für die Umsetzung in Therapiestrategien bedarf es stark intensivierter Anstrengungen. Insgesamt haben die Genomwissenschaften und die damit verbundene Nukleinsäureanalytik sowohl zum Verständnis der molekularen Grundlagen des Lebens als auch zur Aufklärung sehr komplexer Krankheitsbilder und pathologischer Vorgänge enorme Beiträge geleistet.

Die weitere Entwicklung in der medizinischen Versorgung wird durch die Explosion der Kosten belastet, die mit entsprechend aufwendigen Verfahren verbunden sind. Hier muss nicht nur auf die Realisation der Möglichkeiten an diagnostischem und therapeutischem Nutzen gedrängt, sondern auch eine Integration in ein tragfähiges, finanzierbares Gesundheitssystem vorangetrieben werden.

Eine Anwendung entsprechender Technologien in der Forschung kann ebenfalls nur dann in breitem Umfang und auch im akademischen Bereich erfolgen, wenn die damit verbundenen Kosten reduziert werden.

### 1.2 Bedarf

Die Entwicklung der Genom- und Proteomwissenschaften und die Entschlüsselung des Erbgutes stehen noch am Anfang der Entwicklung, ebenso die Realisation des diagnostischen Potentials einer genetischen bzw. gentechnischen Analyse. Die bisher etablierten Verfahren sind meist arbeitsaufwendig und relativ ineffizient, was sich in Kosten und Kapazität z.B. an Informationsgewinn niederschlägt. Wichtigste Neuerung ist die Entwicklung von sog. Oligonukleotid-Arrays, bei denen eine sehr große Anzahl von relativ kurzen Oligonukleotiden definierter Sequenz auf einer festen Matrix (meist Silizium) angekoppelt sind und dadurch für eine parallele Hybridisierung komplementärer Sequenzen im Untersuchungsgut zur Verfügung stehen. Die aufwendige Herstellung und der hohe Preis lassen die Vermarktung als Massenprodukt zum gegenwärtigen Zeitpunkt aber nicht zu.

### 1.3 Anwendungsfelder

Für die in vitro Diagnostik und klinische Diagnostik soll durch deutlich preiswertere Systeme die Anwendung in der Routine möglich werden, z.B. für Infektionskrankheiten (HIV, HBV etc.) und deren Subtypen, für die Onkologie (Tumorfrüherkennung, Tumorklassifizierung, z.B. Typ und Status), und für die Bestimmung einer genetischen Prädisposition.

Für die biologische Grundlagenforschung, insbesondere die Genomik, ist die Erfassung von sehr vielen Messpunkten im untersuchten System, z.B. alle exprimierten Gene wünschenswert. Daraus ergibt sich ein enormer Erkenntnisgewinn in der biologischen Grundlagenforschung (Entwicklungsbiologie, Stammzellkultur, Tissue-engineering, Transplantationsmedizin, Regeneration) ableiten, der auch zu wichtigen Durchbrüchen in der Biomedizin und zu entsprechenden Anwendungen führen wird.

Wie für die Verwendung von DNA-Chips gezeigt wurde (Science 280:1077-1082), kann durch entsprechende biochemische Rahmenbedingungen in der Hybridisierung zwischen Punktmutationen in der Basenabfolge unterschieden werden. Mit dem hier beschriebenen System ist damit ein breit angelegtes Screening möglich, das für forensische Zwecke, z.B. zur Überführung von Straftätern oder zum Verwandtschaftsnachweis eingesetzt werden kann.

Durch diese Erfindung wird auch das rasche, kosteneffektive Analysieren von Lebensmitteln z.B. auf das Vorhandensein bestimmter Gene von pathogenen Keimen oder von genetisch veränderten Organismen hin möglich.

Ebenfalls von großer Bedeutung ist das Screening von medizinischen Produkten. Die Herstellung z.B. von Blutpräparaten ist immer noch mit einem hohen Aufwand für die Sicherheitsvorkehrungen zur Reinheit verbunden. Ein zeit- und kosteneffektives Screening solcher Proben wird mit dieser Erfindung möglich werden, um z.B eine Kontamination mit infektiösem Material zu verhindern (HIV, HBV, HCV etc).

### 2. Stand der Technik

Bei BioChips handelt es sich um miniaturisierte hybride Funktionselemente mit biologischen und technischen Komponenten, z.B. an der Oberfläche eines Trägers immobilisierte Biomaterialien, die als spezifische Interaktionspartner dienen können (z.B. DNA-Oligonukleotide) und eine SiliziumMatrix. Meist sind diese Funktionselemente in Reihen und Spalten angeordnet, man spricht dann von BioChip-Arrays. Da Tausende von biochemischen Funkionselementen auf dem BioChip angeordnet sein können, müssen diese mit mikrotechnischen Methoden hergestellt werden.

Vor allem in den USA wird die Entwicklung von miniaturisierten BioChips mit enormen Mitteln vorangetrieben. Die wichtigsten in diesem Umfeld tätigen Firmen sind im folgenden aufgelistet:

Affymetrix, Beckman Instruments, Blue Chip Biosystems, Caliper Technologies, Cura-Gen, Genometrix, Gene Trace Systems, Hyseq, Incyte Pharmaceuticals, Molecular Tool, Nanogen, Pharmacia, Synteni, Third Wave Technologies, Vysis.

Bisher bekannte BioChips lassen sich nach folgenden Kriterien klassifizieren:
* Nachweisprinzip:
   - Chromatographische Verfahren
   - Interaktion von Analyten mit fester Phase, meist immobilisierter Interaktionspartner (z.B. Hybridisierung von Nukleinsäuren an DNA-Oligonukleotide).
* Detektionsverfahren (optisch, elektrisch).
* Markerbasierte Nachweisverfahren (z.B. Absorption, Fluoreszenz oder Lumineszenz) oder markerfreie Nachweisverfahren (Lichterzeugung zum Reaktionsnachweis).
* Zuordnung des Analyten zu seinem Träger [Festphase] (Array mit mehr als einem immobilisierten Interaktionspartner pro Träger oder Single mit nur einem immobilisierten Interaktionspartner pro Träger).
* Herstellungsverfahren (z.B. Oligonukleotide direkt auf dem BioChip lichtaktiviert synthetisieren, fertig synthetisierte Oligonukleotide spotten, Beads oder Tubes beschichten).
* Trägerarten (Glas-Chips, Kunststoff-Chips, Mikrotiterplatten, Tubes oder Beads).
* Präsentation zur Detektion (seriell, parallel).
* Optische Detektion (seriell im Scanner oder parrallel mit einer CCD-Kamera).

Unter den aufgeführten Firmen verwendet lediglich Affymetrix das Prinzip der Photolithographie für eine "high density" Erzeugung von DNA-Arrays auf einer planaren Oberfläche, wodurch sie die Parallelisierung der Detektion von Oligo-Sequenzen mit Abstand weitesten voran getrieben haben.

### GenChip von Affymetrix Inc., Santa Clara, Kalifornien:

Die Herstellung erfolgt durch in situ Synthese von DNA-Oligonukleotiden auf planaren Chips in hoher Dichte (Juli 98: bis 64.000 unterschiedliche Oligos auf 1 cm²). Das Herstellungsverfahren basiert auf der in der Halbleiter-Industrie verwendeten und optimierten Photolithographie, wobei eine lichtaktivierbare Bindung von Oligos an die Chipoberfläche, sowie an bereits vorhandene Oligos, verwendet wird. Die Herstellungsdauer beträgt aufgrund einer Vielzahl von Verfahrensschritten mehrere Stunden. Der Nachweis erfolgt durch serielle optische Detektion des planaren Chips in einem Fluoreszenzscanner. Die Hybridisierungsdauer der Probe auf einem Chip beträgt ca. 1.5 Stunden. Erste Produkte (Sequenzierchip für Tumormarker p53 Exons 2-11, Brustkrebsgen BRCA1 Exon 11, HIV GeneChip) sind bereits kommerziell erhältlich. Die Kosten liegen zur Zeit im Bereich mehrerer hundert Dollar für einen GenChip, zusätzlich wird noch eine Detektionseinheit benötigt.

Weiterer relevanter Stand der Technik sind W091 /18276, EP-A-0 671 626 und EP-A-0 430 248.

### 3. Gegenstand der Erfindung und damit gelöste Aufgabe

Gegenstand der Erfindung ist ein Verfahren nach Anspruch 1 zur Herstellung eines Trägers für die Bestimmung von Analyten umfassend die Schritte
(a) Bereitstellen eines unmarkierten Trägerkörpers,
(b) Einbringen des Trägerkörpers in eine Syntheseeinheit,
(c) Entnehmen von Informationen über Sequenzen von auf dem Täger zu synthetisierenden Rezeptoren aus einer Datenbank,
(d) Leiten einer Flüssigkeit mit darin enthaltenen Rezeptorbausteinen über den Trägerkörper,
(e) orts- oder/und zeitspezifisches Immobilisieren der Rezeptorbausteine an jeweils vorbestimmten Positionen auf dem Trägerkörper durch Belichten über eine programmierbare Lichtquellenmatrix, und
(f) Wiederholen der Schritte (d) und (e) bis die gewünschten Rezeptoren an den jeweils vorbestimmten Positionen synthetisiert worden sind.

Bevorzugte Ausgestaltungen dieses Verfahrens sind Gegenstand der Ansprüche 2 bis 18. Der Träger ist ein mit biologischen oder chemisch funktionellen Materialien bzw. Rezeptoren (Sonden) oder Bausteinen davon bestückbare oder bestückte Festphase. In dieser Ausführungsform der Erfindung weist der Träger eine mit Vertiefungen, z.B. mindestens einem Kanal und besonders bevorzugt mit einer Vielzahl von Kanälen versehene Oberfläche auf. Die Kanäle sind vorzugsweise Mikrokanäle mit einem Querschnitt von z.B. 10 bis 1000 *µ*m. Die Kanäle können - abhängig von den Oberflächeneigenschaften - Kapillarkanäle, aber auch Kanäle ohne Kapillarwirkung (z.B. aufgrund von Beschichtung mit Teflon) sein. Der Träger ist bevorzugt zumindest teilweise im Bereich der mit Rezeptoren zu bestückenden Positionen bzw. Bereiche optisch transparent. Die mit Rezeptoren zu bestückenden Bereiche des Trägers sind vorzugsweise chemisch und physikalisch miteinander identisch, d.h. sie weisen eine im wesentlichen gleiche Oberflächenbeschaffenheit auf.

Bei der Ausgestaltung des Verfahrens nach Anspruch 17 wird der Trägerkörper in eine Syntheseeinheit eingebracht, umfassend eine programmierbare Lichtquellenmatrix, eine Detektormatrix, Mittel zur Zufuhr von Fluids in den Träger und zur Ableitung von Fluids aus dem Träger, sowie elektronische Steuerungs- und Regelungsmittel, wobei der Trägerkörper zwischen der programmierbaren Lichtquellenmatrix und der Detektormatrix angeordnet wird. Die programmierbare Lichtquellen- bzw. Belichtungsmatrix kann eine Reflexionsmatrix, eine Lichtventilmatrix, z.B. eine LCD-Matrix oder eine selbstemittierende Belichtungsmatrix sein.

Gegenstand der Erfindung ist weiterhin die Verwendung des beanspruchten Verfahrens gemäß den Ansprüchen 19-24.

Gegenstand der Erfindung ist schließlich auch ein System zur integrierten Synthese von Rezeptoren auf einem Träger nach Anspruch 25, umfassend
(a) einen Trägerkörper,
(b) eine Syntheseeinheit umfassend eine programmierbare Lichtquellenmatrix,
(c) Mittel zur Synthese von Rezeptoren durch stufenweisen Aufbau aus Rezeptorbausteinen an jeweils vorbestimmten Positionen des Trägers, und
(d) eine Datenbank zum Entnehmen von Informationen über Sequenzen von auf dem Träger zu synthetisierenden Rezeptoren.

Bevorzugte Ausgestaltungen dieses Systems sind Gegenstand der Ansprüche 26-28.

Eine Ausführungsform der vorliegenden Erfindung stellt ein Verfahren und System zur zyklischen integrierten Synthese und Analyse dar, welches als ISA-System bezeichnet sein soll. Durch diese erfindungsgemäß bevorzugte unmittelbare Kopplung von Synthese und Analyse wird in einem zyklisch verlaufenden Verfahren eine gegenüber dem Stand der Technik deutlich verbesserte Hochdurchsatz-Bestimmung von Analyten ermöglicht. Dabei können die zu analysierenden Substanzen beispielsweise als Bruchstücke oder Fragmente einer größeren Molekülkette vorliegen.

Gemäß einer bevorzugten Ausführungsform der Erfindung wird eine direkte logische Verknüpfung zwischen den Ergebnissen der Analyse eines ersten Trägers und der Synthese des darauffolgend zu erstellenden Trägers bereitgestellt, wobei sich der Informationsgewinn aus einem vorhergehenden Zyklus in einen nachfolgenden Zyklus übertragen lässt. Auf diese Weise wird ein Lernen des Analysesystems schrittweise entwickelt.

Die besagte zyklisch verlaufende Abfolge von Synthese, Sequenzvergleich, Analyse der Vergleichsergebnisse und wiederum erfolgender Synthese von Rezeptoren am Träger kann beliebig oft - bis zum Erreichen eines gewünschten, beliebig zu wählenden Abbruchkriteriums - wiederholt werden.

Durch die Rückkopplung und den damit verbundenen Lernprozess von vorhergehendem Zyklus sind das erfindungsgemäße Verfahren und die Vorrichtung auch für die Erforschung sehr großer und komplexer Analyt-Molekülketten, z.B. zum Sequenzieren individueller Genome, wie dem menschlichen Genom, geeignet. Der Zeitaufwand verbessert sich dabei gegenüber dem Stand der Technik mindestens um das hundertfache, eher um das tausendfache und potentiell um das 10.000-fache.

Das Verfahren kann zur "Neusequenzierung" nicht bekannter Nukleinsäuresequenzen (DNA, cDNA, RNA) einschließlich deren räumlichen Zuordnung, respektive Kartierung eingesetzt werden. Mit diesem Vorgehen ist es möglich, ein individuelles Genprofil jedes Individuums und jeder Spezies zu erstellen, sei es durch Sequenzierung von Teilen des Genoms oder des ganzen Genoms.

Weiterhin kann das Verfahren zur "Resequenzierung" von Nukleinsäuresequenzen eingesetzt werden, d.h. zum Vergleich von bereits bekannten Sequenzen (repräsentiert in Form der Rezeptorsonden) mit unbekannten Sequenzen in der zu untersuchenden Probe. Die bekannten Sequenzen werden dazu gezielt und der Fragestellung entsprechend ausgewählt.

Die beschriebene Resequenzierung erlaubt es dem Anwender, individuelle polymere Rezeptoren vor Ort auf dem erfindungsgemäßen Träger ausgehend von einem neutralen Träger zu erzeugen und anschließend sofort eine Analyse der zu untersuchenden Probe durchzuführen. Durch diese Möglichkeit entsteht eine maximale Variantenvielfalt der Rezeptoren bei minimalem Platzbedarf.

Durch Kombination von Neu- und Resequenzierung, können diagnostische Tests oder Medikamente kurzfristig an die Bedürfnisse eines Individuums angepasst werden.

Als weiteres wichtiges Anwendungsgebiet können außerordentlich flexibel Expressionsmuster analysiert werden. Die entsprechenden Rezeptoren bzw. Polymersonden werden dazu in aller Regel anhand bekannter Sequenzen ausgewählt. Der Einsatz des Verfahrens zur Bestimmung der Genexpression kann auch im Kontext von Hochdurchsatz-Screening erfolgen.

Darüber hinaus sind mit unterschiedlichen natürlich vorkommenden und künstlichen Rezeptorsonden unterschiedliche Ansätze für Screening-Verfahren und den Aufbau und die Analyse von Substanzbibliotheken denkbar. Dies kann z.B. im Zusammenhang mit der Suche nach und der Charakterisierung von pharmakologisch aktiven Substanzen erfolgen.

Die Anwendungsfelder für das erfindungsgemäße Verfahren und die erfindungsgemäße Vorrichtung zur zyklisch integrierten Synthese und Bestimmung von Analyten sind breitgefächert und erstrecken sich prinzipiell auf alle Anwendungen der Analyse wie Gas-Chromatographie, Dünnschicht-Chromatographie,Gel-Elektrophorese,Kapillar-Elektrophorese, Massenspektrometrie etc. Gleiches gilt prinzipiell für alle Anwendungen hochparalleler Festphasen-Analyse.

Die Notwendigkeit, fertige und komplexe polymere Rezeptoren zu lagern, entfällt vollständig. Darüber hinaus gibt es keine physikalische Beschränkung bezüglich der Anzahl und Auswahl der Rezeptoren. Die benötigte Anzahl der Rezeptoren kann über mehrere Reaktionsträger bzw. mehrere Zyklen in einem Reaktionsträger verteilt werden, da die einzelnen Rezeptoren für die logische Auswertung der Vergleichsergebnisse keinen Ortsvorgaben unterliegen.

Gegenstand der vorliegenden Erfindung ist ein neuer "Träger" als Basis für die Verwendung einer vorzugsweise lichtgesteuerten Synthese einzelner Basen (G, A, C und T) oder ganzer Oligonukleotide (Basen- Sequenzen) zur Bildung einer hochparallelen, -planaren und -dichten Anordnung (Array) dieser Oligonukleotide in einer festen Trägermatrix (Chip).

Der neue BioChip, der "opto-fluidische Mikroprozessor", enthält eine Struktur aus Mikrokanälen, vorzugsweise Kapillaren in einem zumindest teilweise durchsichtigen und vorzugsweise flachen Körper. Die flüssigen Einsatzstoffe werden bei der Rezeptorsynthese oder -immobilisierung durch die Kanäle im Träger geführt und binden, lokal aktiviert, an den Kanalwänden. Damit werden die technischen Vorraussetzungen für eine schnelle, effiziente und damit kostengünstige Herstellung geschaffen, was den breiten Einsatz dieser Träger ermöglichen wird. Dichte und Parallelität liegen in der gleichen Größenordnung wie bei Konkurrenztechniken, mit mehreren hunderttausend definierten Oligonukleotiden auf einem Träger. Der Vorteil der neuen Technik liegt in den günstigeren physiko-chemischen Eigenschaften der Strömungs- und Benetzungsvorgänge in den Kanälen im Vergleich mit einer einheitlichen Oberfläche.

Die Chip-Produktion besteht aus der Erzeugung eines vorzugsweise mit Mikrokanälen versehenen Trägerkörpers aus einem geeigneten, lichtdurchlässigen Material sowie dem biochemischen Beschichtungsvorgang vorzugsweise an den Wänden der einzelnen Mikrokanäle, so dass anschließend die polymeren Rezeptoren, z.B. Oligonukleotide, in den Kanälen synthetisiert werden können. Hierbei werden in den einzelnen Kanälen im Träger, mittels Photoaktivierung durch eine geeignete Lichtquelle einzelne Rezeptorbausteine, oligomere Synthone (z.B. Di-, Tri-, Tetra- oder Pentanukleotide) oder ganze Basen-Sequenzen (Oligos) ortsspezifisch angelagert. Dadurch entstehen in jedem Kanal eine Vielzahl an Rezeptor-bestückten Bereichen (spezifische Bindungs- bzw. Hybridisierungsstellen), wobei jeder Bereich aufgrund seiner individuellen Rezeptor-Sequenz-Kombination für die Bindung und anschließende Detektion eines spezifischen Analyten, z.B. eines DNA-Fragments dient. Die Bereiche sind in einer Dimension des planaren Trägers durch die Wände der Kanäle voneinander getrennt und entlang der einzelnen Kanäle wird zwischen zwei benachbarten Bereichen bei der photoaktivierten Bindung ein entsprechender Freiraum gelassen. Es entsteht ein hoch paralleler, hoch integrierter Array von spezifischen Rezeptoren. Aufgrund der Möglichkeit des Multiplexens von Oligo-Sequenzen und parallelen Kanälen (Details siehe Kapitel 5) ist eine Reduktion der Herstellzeiten auf 1/4 bei der Verwendung einfacher Basen, 1/8 bei Dinukleotiden und auf 1/16 bei Trinukleotiden durch entsprechendes Multiplexing der Oligos (Einsatzstoffe) und der zu benetzenden Kanälen möglich. Damit wird auch eine flexible Anpassung an Kundenwünsche, der "massgeschneiderte" BioChip, möglich. Diese systematische Beschleunigung ist in planaren Systemen (planaren Chips) nicht möglich.

Für die Analyse wird das Untersuchungsmaterial (z.B. DNA, RNA in Lösung) durch die Kanäle geführt und erhält Gelegenheit zur Bindung an die Rezeptoren, z.B. durch Hybridisierung an komplementäre Stränge, sofern diese vorhanden sind. Zur Detektion und Auswertung der jeweiligen Analytbindung, z.B. einer DNA-Hybridisierung, werden vorzugsweise hochauflösende, parallele CCD- Chips verwendet. Die Bindung des Analyten an den immobilisierten Rezeptor wird durch geeignete aus dem Stand der Technik bekannte signalgebende Gruppen, z.B. Lichtemittierende Gruppen, durchgeführt. Es sind aber auch neue Detektionsverfahren anwendbar. Bei der Detektion kann auf optisch abbildende Linsensysteme verzichtet werden, wenn die Größe der Kanäle so gewählt wird, dass jeder Messpunkt eine ausreichende Anzahl Pixelelemente des Detektors, z.B. eines CCD-Chips überdeckt. Durch diese direkte Nutzung (keine Optik) hoch paralleler CCD-Matrix Chips mit einer großen Anzahl (derzeit 16 Mio. Pixel pro 1 cm²; Stand der Forschung: 80 Mio. Pixel pro 1 cm²) an Pixeln (optischen Sensoren) ist es möglich eine Vielzahl von Lichtsignalen parallel zu detektieren (siehe BioScanner der Firma Genometrix). Damit wird versucht, auch bei der Detektionseinheit statt teurer optischer Anordnungen auf ein in großer Stückzahl und zu niedrigen Preis gefertigtes High-Tech Produkt zurückzugreifen.

Durch die Erfindung werden somit wesentliche Anforderungen in der DNA-Analytik abgedeckt, nämlich simultane Bestimmung einer Vielzahl an DNA-Sequenzen (erreicht durch hoch integrierte, miniaturisierte Träger und eine hochauflösende optische Detektion), Bereitstellung kostengünstiger Tests (Multiplexing in der Produktion, billige "Disposable"- Träger zum Beispiel aus Spritzguss, schnelle Synthese bei der Produktion), eine schnelle Durchführung bei der Analyse aufgrund kleinerer Volumina und günstiger Benetzungsvorgänge, Reduktion der Einsatzstoffe durch die Strömungsgeometrie des Träger etc.), schnelle Auswertung (erreicht durch die parallele optische Auswertung in planarer Anordnung [DNA-Chip Array]), ein kostengünstiges Analysesystem (erreicht durch den Verzicht auf teure, mikrosystemtechnische und optische Komponenten) und die Sicherstellung der Qualität sowohl bei der Produktion, als auch bei der Analyse (erreicht durch definierte Strömungsvorgänge im Träger).

Die Verwendung der Photoaktivierung von chemischen Reaktionen im Bereich der Träger-Synthese kommt insbesondere in Kombination mit der Technologieplattform des opto-fluidischen Mikroprozessors zusammen mit einer programmierbaren Lichtquellenmatrix zum Durchbruch, da hierdurch die Produktionskosten für einen einzelnen Träger, bei gleichzeitiger Qualitätsverbesserung, um den Faktor 10-100 reduziert werden können. Dadurch wird zum ersten mal eine kostengünstige, massiv parallele, hoch integrierte und gleichzeitig einfach miniaturisier- und automatisierbare DNA-Chip-Technologie zur Verfügung gestellt.

Trotz der komplexen Datenauswertung bedarf es nur eines Minimums an unterschiedlichen Hardware-Komponenten, da die Trägerkörper, die entweder pro Zyklus oder auch nur bei Verschleiß gewechselt werden müssen, zunächst - vor Beginn der Rezeptorsynthese - alle identisch sind. Alle Individualität ergibt sich erst aus der spezifischen Rezeptorsynthese und aus der schrittweise durch die Analyse gewonnene Information, die nach dem Synthese/Analysezyklus wieder in Information überführt wird, so dass die Individualität, d.h. die kennzeichnenden Merkmale der biologischen/chemischen Materie wiederum nur in Form elektronischer Daten vorliegen.

### 4. Grundzüge des Lösungsweges

Der prinzipielle Lösungsweg in diesem System basiert auf der schrittweisen biochemischen Synthese von Rezeptoren an die Oberflächen einer Vielzahl von Kanalwänden auf einen Träger. Diese Kanäle sind auf dem Träger, z.B. einem kleinen planaren Chip, angeordnet. Die Synthese erfolgt mit den entsprechenden Basen oder mehrbasigen Oligonukleotiden (Basen-Sequenzen) über eine lichtaktivierte ortsspezifische Bindung. Die Benetzung dieser spezifisch "gelabelten" Kanäle mit den zu untersuchenden DNA-Analyten und der anschließenden Detektion der Bindungsreaktion über geeignete signalgebende Gruppen schließt einen Verfahrenszyklus ab.

### 4.1 Mikrostruktur als Trägermatrix

Die Träger-Synthese umfasst die Bereitstellung des Trägerkörpers, der vorzugsweise aus einem geeigneten, lichtdurchlässigen Material besteht, sowie dem biochemischen Erzeugen von Rezeptoren an den Wänden der einzelnen Kanäle. Die spezifische Synthese der Rezeptoren kann entweder direkt bei der Herstellung des Trägerkörpers oder erst beim Anwender erfolgen.

Für die Trägerkörper können unterschiedliche Materialien (z.B. Glas, Silizium, Keramik, Metall oder Kunststoff) verwendet werden. Wichtig ist, dass die Wände der Kanäle sowohl für die Anregungswellen bei der lichtaktivierten Synthese sowie die Lichtwellen (ggf. Anregung und Reaktionssignal) bei der anschließenden Detektion (Analyse) gut durchlässig sind. Je nachdem welches Material eingesetzt wird, müssen die Wände der Kanäle mit einem reaktionsfähigen Material beschichtet werden, damit die Rezeptoren oder Rezeptorbausteine an der Oberfläche binden können.

Die Geometrie der Träger entspricht beispielsweise einer "Scheckkarte", wobei die Größe der von den Kanäle bedeckten Fläche von dem zur Detektion verwendeten CCD-Chip bestimmt wird. Für die Kanäle im Träger sind unterschiedliche Herstellverfahren einsetzbar. Hierbei ist der Einfluss der Querschnittsgeometrie der Kanäle zu berücksichtigen, welcher großen Einfluss auf die entstehenden strömungstechnischen Kräfte und die Möglichkeit der Reinigung der Kanäle hat. Als Herstellverfahren kann man zum Beispiel Laser, Fräsen, Ätztechniken oder Spritzguss verwenden.

Bei der Anordnung der Kanäle in der Ebene sind die folgenden Aspekte zu berücksichtigen: Verwendet man eine Vielzahl an parallelen Kanälen, so kann man die Zeiten bei der Synthese minimieren, allerdings ist die Benetzung bzw. Befüllung des einzelnen Kanals entsprechend komplex. Hat man im anderen Extrem nur einen einzigen langen Kanal, so ist die Synthese entsprechend langsam, da das Multiplexing von Kanälen zu Basen oder ganzen Oligos nicht verwendet werden kann und alle Vorgänge nur seriell nacheinander ablaufen können. Der Vorteil nur eines Kanals liegt in der Analyse, wo die Probe an jedem Messpunkt in allen Kanälen vorbeiströmt.

### 4.2 Synthesezyklus im Träger

In einem Trägerkörper werden die zur Beschichtung mit Rezeptoren vorgesehenen Positionen (Reaktionsbereiche) durch Kanäle aus Behältern über Zuleitungen, Ventile und Fittings mit einem oder mehreren Fluiden befüllt. Mit Hilfe einer aus der deutschen Patentanmeldung 198 39 254.0 bekannten Lichtemissions/Detektionseinrichtung, die vorzugweise eine programmierbare Lichtquellen- bzw. Belichtungsmatrix darstellt, wie sie in der deutschen Patentanmeldung 199 07 080.6 beschrieben ist, können ausgewählte Positionen bzw. Bereiche des Trägers belichtet und auf diese Weise die individuelle Synthese von Rezeptoren gesteuert werden, wobei der Träger in diesem Zusammenhang einen opto-fluidischen Mikroprozessor darstellt. Anstelle einer Belichtung ist auch eine individuelle fluidische Aktivierung der ausgewählten Reaktionsbereiche möglich. Nach Abschluss der Reaktion werden die Reaktionsbereiche gespült und neu gefüllt, wonach sich wiederum ein Aktivierungszyklus anschließt. Der Verlauf der Rezeptorsynthese kann mittels geeigneter Detektionseinrichtungen verfolgt und gesteuert werden.

Sobald die Synthese der Rezeptoren abgeschlossen ist, werden die Reaktionsbereiche gereinigt und stehen dann für ein Analytbestimmungsverfahren zur Verfügung.

### 4.3 Nukleinsäureanalytik mittels Oligo-chips - Grundprinzip

Wie bereits für mehrere Anordnungen gezeigt (z.B. Molekular Medicin Today, 9/97, S. 384-389; Trends in Biotechnology, 11/97, S. 465-468) kann die Hybridisierung von Nukleinsäuresträngen an eine meist kurze komplementäre Sequenz, ein sog. Oligonukleotid oder Oligo, für die Sequenz-Analyse verwendet werden. Dazu werden hochdichte Anordnungen synthetischer Oligonukleotide auf einer festen Matrix erzeugt und erlauben multiple parallele Hybridisierungsexperimente. Führendes Verfahren (August 98) ist eine photolithographische und damit lokale Aktivierung von Synthese-Vorstufen. In Anlehnung an die aus der Mikroelektronikherstellung entlehnte Technik werden die parallelen Anordnungen als Chips bezeichnet.

Durch eine massive Erhöhung der Zahl an Reaktionsbereichen ('Messpunkten'), d.h. definierten Oligos an definiertem Ort, wird eine enorme analytische Kapazität geschaffen.

Die zu untersuchende Probe enthält normalerweise DNA oder RNA. Diese muss eventuell isoliert und in einem Amplifizierungsschritt (z.B. PCR) vermehrt werden und erhält dabei eine Markierung, z.B. ein Farbstoff-, Fluoreszenz- oder Lumineszenzlabel.

Durch ausreichend viele Rezeptor-bestückte Bereiche (Reaktionsbereiche) ist auch eine Sequenzierung eines DNA Moleküls möglich (Sequencing-by-Hybridization SBH, siehe BioTec 3/98, S. 52-58), andere Anwendungen zeigen die Bestimmung von Punkmutations-Polymorphismen (d.h. Unterschiede zwischen Individuen in einzelnen Basen in einem definierten DNA Abschnitt) und erlauben u.a. eine Identifizierung von solchen Polymorphismen bei hunderten von Probanden parallel (Science 280, 5/98, S. 1077-1082).

Erstmals wird auch die Untersuchung von ganzen Genomen und des Gen-Expressions- Status ganzer Zellen möglich (z.B. Proc.Nat.Acad.Sci.USA 95, 3/98, S. 3752-3757).

Die hier beschriebene Erfindung lässt demnach die Anwendung einer Vielzahl von etablierten Verfahren zur Untersuchung von Nukleinsäuren und genetischem Material zu. Damit ist gleichzeitig ein starker Anstieg solcher Anwendungen und damit ein enormer wissenschaftlicher Fortschritt verbunden, da erwartet wird, dass der opto-fluidische Mikroprozessor eine solche Technologie flexibler als die vorhandenen Verfahren und zu deutlich niedrigeren Kosten bereitstellt.

### 4.4 Lichtaktivierte Synthese von Oligonukleotiden und Peptiden auf dem Träger

Beim Aufbau von Rezeptoren auf dem Träger werden in den einzelnen Bereichen mittels Photoaktivierung durch eine geeignete Lichtquelle Rezeptorbausteine, z.B. einzelne Basen (G, A, C, T) oder Oligonukleotid-Sequenzen (vorzugsweise etwa 2 bis 4 Basen lang) ortsspezifisch angelagert. Die Kanäle werden sequentiell mit den Synthesebausteinen, z.B. G, A, C und T, gefüllt und entlang der Kanäle ortsspezifisch mit hochauflösendem Licht bestimmter Wellenlänge und Intensität bestrahlt. Zwischen den Beschichtungszyklen werden die Kanäle entsprechend gespült, um nicht gebundene Rezeptorbausteine zu beseitigen.

Hierdurch entstehen in jedem Kanal eine Vielzahl an Reaktionsbereichen (spezifische Bindungs- bzw. Hybridisierungsstellen), wobei jeder Reaktionsbereich aufgrund seiner individuellen Rezeptor-Sequenz für die Bindung und anschließende Detektion eines spezifischen Analyten, z.B. eines DNA-Fragments dient. Die Reaktionsbereiche sind in der einen Dimension des planaren Trägers durch die Wände der Kanäle voneinander getrennt und in der zweiten Dimension, entlang der einzelnen Kanäle, wird zwischen zwei benachbarten Reaktionsbereichen bei der Photoaktivierung ein entsprechender Freiraum gelassen.

Die Photolithographie kann auch weiterhin für die lichtaktivierte Bindung der Rezeptorbausteine verwendet werden. Es können aber auch andere Verfahren eingesetzt werden.

Besonders bevorzugt wird ein Belichtungsverfahren unter Verwendung einer programmierbaren Lichtquellenmatrix, z.B. einer selbstleuchtenden Lichtquellenmatrix, einer Lichtventilmatrix oder einer Reflexionsmatrix durchgeführt, deren Matrixpunkte bzw. Lichtquellenelemente gezielt steuerbar sind, insbesondere hinsichtlich der Intensität und gegebenenfalls Farbe des Lichtes. Mit einer solchen Matrix können also jeweils benötigte zweidimensionale Belichtungsmuster auf einfache Weise, insbesondere rechnergestützt erzeugt werden. Die bevorzugte Photoaktivierung der Oligos bei der Herstellung des Trägers erfolgt direkt durch die Belichtungsmatrix. Die hierfür benötigte Wellenlänge von beispielsweise 365 nm (oberer UV-Bereich nahe dem sichtbaren Licht) lässt sich mit allen Varianten der programmierbaren Lichtquellenmatrix steuern.

Auf entsprechende Weise können auch Rezeptoren aus Aminosäureoder/und Peptidbausteinen aufgebaut werden.

### 4.5 CCD-Chip-Detektion der spezifischen Nachweisreaktion

Wie beschrieben soll die Bindung eines DNA-Analyten direkt oder indirekt zu einem nachweisbaren Signal, z.B. zu einem Lichtsignal führen. Dies kann beispielsweise durch Absorbtion, ein Anregungslicht (Fluoreszenz) oder durch Photonenemission (Lumineszenz) erfolgen. Zur Signaldetektion wird vorzugsweise ein CCD-Chip verwendet, der vorzugsweise direkt unter dem Träger plaziert wird. Die Anregungslichtquelle wird vorzugsweise über dem Träger plaziert und es wird entsprechend im Durchlichtverfahren gemessen.

Jedes Lichtsignal kann auf dem CCD-Chip erfasst werden, und zwar nach Intensität und bei Bedarf auch nach Wellenlänge (Farbe) differenziert. Das aufgenommene Spektrum kann qualitativ oder quantitativ ausgewertet werden. Zudem lässt die Unterscheidung von Wellenlängen und Intensitäten auch eine Unterscheidung von Signalquellen zu.

Die Anregungslichtarten für das Nachweisverfahren müssen je nach Anforderungen monochromatisch (z.B. Laserlicht für Fluoreszenzanregung) oder heterogen (z.B. Weißlicht für Absorptionsmessung) gewählt werden.

### 5. Verbesserungen und Vorteile gegenüber vorhandenen Systemen

Durch die neuen Träger werden die nachfolgend aufgeführten Nachteile von maskenbasierten Photolithographieverfahren oder dem in situ-Spotten überwunden.
* Das Prinzip der flächigen Benetzung der gesamten Chipoberfläche mit Fluid erlaubt keinerlei Multiplexing in der Produktion. So erhöht sich die Zahl der Herstellungszyklen für 20 Basen lange Oligos bei einer Verwendung von Dinukleotiden (4² = 16 Möglichkeiten) von 4 x 20 = 80 Hybridisierungsschritten auf 16 x 10 = 160, was eine Verdoppelung bedeutet. Das Gleiche gilt natürlich auch für die zwischengelagerten Waschzyklen.
* Die Synthese der photoaktivierbaren Basen an die planare Chipoberfläche, ebenso wie die benötigten Waschschritte bei der Chipherstellung sind, außer durch platz- und handhabungsintensive Tauchvorgänge (Chip wird in Flüssigkeit getaucht) oder flüssigkeitsintensive Spülvorgänge entlang der Oberfläche (z.B. Zentrifugationsprinzip aus der Halbleitertechni), nicht realisierbar, was von der Geräteentwicklung her gesehen ein sehr großes Miniaturisierungs- und Automatisierungshemmnis darstellt.
* Bei der anschließenden DNA-Sequenz-Detektion ist eine gleichmäßige Verteilung der Probe auf der Chipoberfläche aufwendig (keine einfachen und damit zuverlässigen Mischverfahren möglich) und es Bedarf einer entsprechend großen Menge an Proben-Fluid. Die Suche nach einem seltenen Ereignis in der Probe ist nicht möglich, da ein ausreichender Kontakt aller Probenbestandteile mit allen spezifischen Messpunkten nicht gewährleistet werden kann.

### 5.1 Reduktion der Produktionszeiten durch Multiplexing beim Synthetisieren

Der wesentliche Fortschritt der neuen Träger liegt in der Möglichkeit der drastischen Reduktion der Herstellzeiten bei der individuellen Synthese der Rezeptor-bestückten Träger durch ein entsprechendes Multiplexen zwischen Rezeptorbausteinen als Einsatzstoffen und den Kanälen.

Für die ortsspezifische Erzeugung einer Vielzahl an unterschiedlichen Rezeptorsequenzen, z.B. Basen-Sequenzen einer bestimmten Länge (z.B. 20 Basen) auf einer planaren Oberfläche mittels örtlich hochauflösender Photoaktivierung benötigt man in jeder Ebene (Rechenbeispiel: 20 Basen in jeder Basen- Sequenz) des DNA-Chip-Arrays 4 (bedingt durch die vier verschiedenen Basen) Synthesezyklen. Für 20 Basen- Ebenen sind es folglich 4 x 20 = 80 Zyklen. Verwendet man auf der gleichen Oberfläche Dinukleotide (2 Basen) so entstehen 2 Ebenen auf einmal, allerdings sind für diese zwei Ebenen 4² = 16 Synthesezyklen notwendig. Für 20 Ebenen werden folglich 10 x 16 = 160 Synthesezyklen anstelle von 80 Zyklen benötigt, was eine Verdoppelung der Produktionszeiten bedeutet. Bei der Verwendung von Trinukleotiden (3 Basen) verstärkt sich dieser Effekte auf mehr als die fünffache Anzahl an Zyklen. Somit ist bei einer einfachen planaren Oberfläche die Verwendung von einzelnen Basen als die schnellste Möglichkeit zur photoaktivierten DNA-Chip Erzeugung gegeben. Es besteht keine Möglichkeit die Anzahl an Synthesezyklen zu reduzieren.

Bei der Synthese des opto-fluidischen Trägers besteht im Unterschied hierzu die Möglichkeit, die Einsatzstoffe, sprich die Basen oder die unterschiedlichen Varianten der Di- (4² = 16 Kombinationen) oder Trinukleotide (43 = 64 Kombinationen) auf verschiedene Kanäle zu verteilen. D.h., zumindest in den unteren - "trägernahen" - Ebenen wird je Kanal nur immer eine Base bzw. eine der möglichen Basen-Sequenzen eingebracht. Je nach Festlegung der insgesamt zu erzeugenden Basen-Sequenzen in den Kanälen des Trägers kann es sein, dass in den oberen Ebenen dieses Prinzip teilweise aufgehoben werden muss, sprich für eine Basen-, Di- oder Trinukleotid- Ebene muss mehr als eine Base oder Oligo durch einen der Kanäle fließen. Dadurch erhöht sich auch hier die Anzahl der Synthesezyklen ggf. wieder etwas. Insgesamt bleibt jedoch eine sehr große Reduktion der Herstellzeiten auf theoretisch 1/4 der Zyklen bei einfachen Basen, 1 /8 der Zyklen bei Dinukleotiden und auf 1 /16 der Zyklen bei Verwendung von Trinukleotiden als Einsatzstoffe bei der RezeptorSynthese (und so weiter bei längeren Oligos). Die Anzahl der für einen spezifischen Träger benötigten Zyklen ist für jeden Träger individuell und kann nur als statistischer Mittelwert angegeben werden, wenn die Anzahl an Reaktionsbereichen auf bzw. in dem Träger, die Anzahl an parallelen Kanälen und die Länge der auf dem Träger zu synthetisierenden Oligos vorgegeben ist. Die Optimierung der Synthesezeiten eines Trägers soll mittels eines zu entwickelnden Softwaretools (z.B. CAMS Computer Aided Multiplexing Synthesis) erfolgen, welches in die Steuerung des zu entwickelnden Analysesystems bzw. in den angekoppelten Rechner integriert wird.

### 5.2 Reduktion der Einsatzstoffe und Qualitätssicherung

Die Verwendung von Kanälen reduziert die benötigte Fluidmenge und erhöht gleichzeitig die Qualität sowohl bei der Träger-Synthese, als auch bei der anschließenden Detektion einer Probe, im Vergleich zur Verwendung einer einfachen Fläche sehr stark. So ist das gleichmäßige Benetzen von Kanälen strömungstechnisch sehr einfach, verbraucht wenig Fluid und ist daher sehr leicht miniaturisier- und automatisierbar. Dies gilt insbesondere auch für die benötigten Waschvorgänge der Kanäle mit einer ausreichenden Qualität.

Durch die Wände der Kanäle, welche im Prinzip den Zwischenraum zwischen zwei Reaktionsbereichen im Träger-Array bedecken, wird das benötigte Fluid bereits um 50% reduziert. Dies gilt sowohl für das Beschichten des Trägers in der Produktion, das Synthetisieren der Rezeptoren als auch für den "Probenauftrag" für die Analyse. Eine weitere Reduktion der Fluidmengen erfolgt durch die gute Benetzung der Kanalwände durch ein durchströmendes Fluid und vor allem durch die effektiven Waschvorgänge, welche zum Beispiel durch "reinigende" Gasblasen in den Kanälen stark verbessert werden können. Eine gute, statistisch ausreichende Verteilung der Probe auf einer Fläche ist dagegen nur mit einer sehr großen Probenmenge realisierbar.

Ein weiterer Vorteil der Kanäle liegt in den kürzeren Zykluszeiten, welche durch die kleineren Fluidvolumina und die damit verbundenen schnelleren chemischen Reaktionen und Abläufen entstehen. Dies hat sowohl kürzere Synthese- als auch Hybridisierungszeiten zur Folge.

Zusätzlich wird hierdurch eine deutliche Fehlerreduktion sowohl bei der Produktion wie bei der Detektion erzielt, was die Zahl der auswertbaren Messungen pro Material- und Zeiteinsatz weiter erhöht und die Grundlage für eine Qualitätssicherung bildet, welche auf genau definierbare und reproduzierbare Strömungsvorgänge aufbaut.

Die einfache Miniaturisierung und Automatisierung der Abläufe in den neuen Trägern bilden die Basis für eine einfache Miniaturisierung und Automatisierung des gesamten neuen Analysesystems, welches auf den Trägern aufbaut.

### 5.3 Dreidimensionale Reaktionsoberflächen

Durch eine geeignete Auslegung der Querschnittsgeometrie der einzelnen Kanäle lässt sich die nutzbare Reaktionsoberfläche vergrößern. Die Größe dieser Fläche ist für die Anlagerung der Oligos bei der Produktion ebenso von Bedeutung wie für die Anlagerung der vorbeiströmenden DNA-Fragmente aus der Probe und der daraus resultierenden Intensität der Lichtsignale bei erfolgter Hybridisierung.

So hat ein rechteckiger Kanal, gleiche Höhe wie Breite vorausgesetzt, bei einer Nutzung der Wände und der Decke die vierfache Reaktionsoberfläche bei einer identischen Grundfläche, sprich dem gleichen Platzbedarf in den zwei Dimensionen eines planaren Trägers. Selbst wenn man die Kanäle, aus strömungstechnischen Anforderungen heraus, innen rund auslegt (zum Beispiel bessere Reinigungsmöglichkeiten durch Gasblasen im Kanal), bleibt noch eine etwa dreifache Reaktionsoberfläche im Vergleich mit einer planaren Oberfläche. Durch die Nutzung dieser dreidimensionalen Strömungsgeometrie kann der Einsatzstoffbedarf (Produktion und Analyse) weiter reduziert werden.

Ein anderer Effekt lässt sich ebenfalls durch die Querschnittsgeometrie der Kanäle beeinflussen: Die Lichtbrechung am Übergang vom Innenraum der Kanäle in das umgebende Material des Trägers. So hat jede Krümmung entweder einen fokussierenden oder streuenden Einfluss auf die Ausbreitungsrichtung des Lichtes. So kann man beim Träger durch eine entsprechende Wahl der Ober- und Unterseite der Strömungskanalgeometrie die Lichtwege optimieren.

### 5.4 Parallele CCD-Chip Detektion

Das Messen der Lichtsignale aller Reaktionsbereich des Trägers "auf einmal" nutzt das ständig wachsende Potential der hochauflösenden CCD-Kamera Chips. Diese erlauben die Detektion aller Lichtsignale zum Reaktions- bzw. Hybridisierungsnachweis in einem einzigen Messvorgang. Hierfür stellen aktuelle Farb-CCD-Chips auf einer Fläche von 40 x 40 mm etwa 3000 x 3000 Pixel mit einer Pixelgröße von etwa 10x10 *µ*m zur Verfügung. Der Stand der Forschung ist bereits bei entsprechenden CCD-Chips mit ca. 4000 x 6000 Pixeln. Die Signaldetektion erfolgt in Bruchteilen einer Sekunde für alle Pixel synchron. Damit ergibt sich auch für die beschriebene Applikation der CCD-Chip Technologie ein großes Wachstumspotential und die parallele Detektion von 10⁶ individuellen Reaktionsbereiche im Träger ist technisch machbar. Dadurch werden die zeitaufwendigen Scann-Vorgänge herkömmlicher Systeme vermieden und die reine Messzeit reduziert sich auf ein Minimum, welche im Verhältnis zu anderen Verfahrensschritten völlig bedeutungslos wird.

Das Bearbeiten der anfallenden Datenmengen ist, durch die Entwicklung der Leistungsfähigkeit bei gleichzeitigem Preisverfall von modernen Rechnersystemen, problemlos möglich.

### 5.5 Direktdetektion ohne Optik

Die direkte Detektion der Lichtsignale, ohne Optik, durch einen CCD-Chip hat den Vorteil einer wesentlich niedrigeren Energiemenge, welche das Licht für eine fehlerfreie Detektion benötigt. Eine solche Anordnung soll - in einem anderen Zusammenhang untersucht - lediglich 10% der Anregungslichtmenge einer vergleichbaren Anordnung mit einer Optik verbrauchen. Anders ausgedrückt, die Optik schluckt 90% der Lichtenergie. Durch die geringere Lichtintensität werden unerwünschte Streulichteffekte im - die Kanäle umgebenden - Träger, ebenso wie eine eventuelle notwendige Kühlung der verwendeten Lichtquelle, stark reduziert. Außerdem bedeutet der Wegfall einer Optik eine große Platzersparnis sowie eine Verringerung der Herstellkosten für die Detektionseinheit.

Aufwendige Bewegungseinrichtungen für den Träger oder die Detektionseinheit, wie sie in Scannern notwendig sind, entfallen ebenfalls völlig. Die vorgegebenen Abmessungen der CCD-Chips (mehrere cm²) ermöglichen die Verwendung einer sehr großen Zahl an parallelen Kanälen (mehrere 100) mit einer moderaten Kanalgröße (im 10-100 µm Bereich).

### 5.6 Disposable Träger

Die Träger können als einfache Disposables (Einweg-Chips) ausgeführt werden. Prinzipiell sind sowohl Glas-, Silizium-, Metall-, Keramik- oder Kunststoff-Chips (kostengünstige Spritzguss-Verfahren) sowie andere Ausführungen möglich.

Die Biochips anderer Technologien sind ebenfalls als Disposable für wenige Messungen ausgelegt. Hier spricht jedoch der aufgrund der aufwendigen Herstellung der Chips sehr hohe Preis meist gegen das Wegwerfen des Chips nach nur einer oder ein paar Messungen.

### 5.7 Flexibilität der Anwendung

Die schnelle und kostengünstige Produktion ermöglicht eine Vielfalt von individuellen Anwendungen, bei denen z.B. unter Berücksichtigung von Sequenz- und Gendatenbanken im Internet gezielt Oligonukleotid-Arrays synthetisiert werden.

Durch die Verwendung eines einzigen, vielfach gewundenen oder spiralförmigen Kanals könnte eine Hybridisierung im (langsamen) Durchfluss etabliert werden, die auch die Detektion von seltenen Ereignissen (z.B. selten exprimierte Gene) ermöglicht. Damit würde ein chromatographisches Prinzip in die DNA Array Technologie eingeführt werden.

Durch die Verwendung von Di-, Tri- oder längeren Oligonukleotiden als Synthesebausteinen ist eine weitere Reduktion der Herstellungszeiten erreichbar. Vor allem für einfachere Arrays können Syntheseeinheiten direkt beim Kunden zur Anwendung kommen und damit die Zusammensetzung des Arrays endgültig individualisieren.

Die große Flexibilität der Technologie ist auch im Hinblick auf die Erkenntnis von Bedeutung, dass sich die Gene einzelner Individuen sehr stark unterscheiden, so dass man keinen generellen Genkatalog für alle Spezies anlegen kann. Der Träger eröffnet hier die Möglichkeit, zum Beispiel in einem ersten Messzyklus die Basisdaten, wie sie im Internet - frei zugänglich oder nur spezifisch für den Systemkunden - bereitgestellt sind mit den individuellen Unterschieden eines Patienten abzugleichen und aus den Ergebnissen einen entsprechenden zweiten DNA-Array zu bilden, welcher die eigentlichen Tests auf das Individuum angepasst durchführt.

Die erfindungsgemäße Lösung kann auch für die Synthese von Peptidsequenzen in den Kanälen verwendet werden. Damit würden für eine Vielzahl von Anwendungen hoch komplexe und zugleich kostengünstige Peptidarrays bereitgestellt werden.

### 6. Überblick über einige Aspekte der Erfindung

### 6.1 Ausführungsvarianten des Trägers

Bei der Gestaltung ebenso wie bei der Fertigung der Träger gibt es eine Vielzahl von Ausführungsvarianten. Bei der Anordnung der Kanäle im Träger über der Fläche der Detektionseinheit ist die Verwendung nur eines Kanals ebenso denkbar wie die Anordnung einer Vielzahl an parallelen Kanälen. So sind auf einer Fläche von 25 x 37 mm fertigungstechnisch problemlos 500 Kanäle (Stand der Technik: 500 parallele Kapillaren mit einem Durchmesser von 900 nm) mit einer Länge von 37 mm und jeweils etwa 750 Reaktionsbereichen anzuordnen. Die gleiche Anzahl an Reaktionsbereichen (500 x 750 = 375.000) ließe sich auch in einem einzigen schlangenförmigen Kanal mit etwa 20 m Länge unterbringen.

Der Vorteil nur eines Kanals liegt in der Präsentation der Probe an allen Messpunkten des Arrays und ist daher für die Suche nach seltenen Bestandteilen besonders geeignet. Ein Vielzahl an parallelen Kanälen hat den Vorteil, dass sich die Produktionszeiten bei der Träger-Synthese durch das Multiplexen von Einsatzstoffen und Kanälen sowie alle Strömungsvorgänge minimieren lassen. Deshalb ist diese Kanalanordnung für die Träger-Synthese sowie alle Analysen mit einer ausreichenden Anzahl an Kopien jedes Analyten in der Probe zu bevorzugen.

Um beide Vorteile in einem Träger zu nutzen ist es möglich die Einsatzstoffe bei der Träger-Synthese mittels paralleler Fittings am Zugang zu den Kanälen einzubringen, obwohl der Kanal von der Probeneingabe an nur aus einem einzigen, langen Mikrokanal besteht. Dieser Effekt kann auch durch die Integration von Ventilen in den Träger oder die umgebenden Gerätekomponenten erfolgen. So hat die Firma Biacore fluidisch angesteuerte Ventile in einem zweiteiligen Spritzguss-Chip durch eine Membran realisiert, welche von unten in die Kanäle auf der Oberseite des Chips drückt und so die Kanäle verschließt.

Als Anordnung für die Kanäle über der Detektorfläche ist eine Vielzahl an Strukturen und Mikrokanalverläufen möglich. Für eine hohe Parallelität der fluidischen Vorgänge sind beispielsweise parallele oder "snakeförmige" Strukturen naheliegend. Die Aufteilung der Kanäle sollte hierbei nach dem Dualprinzip erfolgen, wo aus jedem Kanal zwei neue entstehen und alle Kanäle gleich lang sind. So erreicht man nach 10 Teilungen bereits 2¹⁰ = 2048 Kanäle. Spiralförmige Anordnungen haben den Vorteil, das sie weniger turbulente Strömungsvorgänge aufweisen und besser zu reinigen sind. Ihr großer Nachteil liegt in der Zu- bzw. Abführung, welche in der dritten Dimension nach oben oder unten erfolgen muss, was fertigungstechnisch und optisch eher ungünstig ist.

Als Material für die Träger ist beispielsweise Glas, Silizium, Keramik, Metall oder/und Kunststoff möglich. Der Aufbau kann in zwei Schichten erfolgen, welche zum Beispiel durch Kleben oder Bonden aneinandergefügt werden können oder nicht. Die Struktur der Kanäle kann hierbei entweder nur in die eine, oder aber in beide Seiten bzw. Hälften eingebracht werden. Hierfür sind als Fertigungsverfahren u.a. Laser oder Präzisionsfräsen verwendbar. Besonders kostengünstig ist Spritzguss, welcher in einer ausreichenden Qualität gefertigt werden kann. Weitere Verfahren sind die LIGA-Technik oder Heißformen.

### 6.2 Träger - Synthese zu

Für die Synthese der individuellen Fänger-Rezeptoren, z.B. Oligos, auf den Reaktionsbereichen im Träger-Array gibt es prinzipiell zwei Möglichkeiten. Der Kunde ersteht fertige Träger vom Hersteller mit einer vorgegebenen Auswahl an immobilisierten Basen- Sequenzen, oder er synthetisiert sich in einer Syntheseeinheit seine selbstgewählten Sequenzen auf ungelabelte Träger. Informationen über entsprechende Sequenzen können zum Beispiel aus Datenbanken im Internet entnommen werden, die hier frei oder auch speziell durch den Träger- Hersteller bereitgestellt werden.

### 6.2.1 Syntheseeinheit

Die Syntheseeinheit besteht aus einer geeigneten Lichtquelle, welche die Reaktionsbereiche im Träger-Array bei der Synthese der Rezeptoren, z.B. Basen oder Basen-Sequenzen, an der Trägeroberfläche bzw. den Kanalwänden ortsspezifisch hochgenau und exakt auflösend bestrahlt. Wie bereits unter 4.4 erwähnt, kann die Belichtung mittels einer programmierbaren Lichtquellenmatrix erfolgen. Verwendet werden kann auch eine Photolithographie- Einrichtung, wie sie in der Halbleiter- Chip-Produktion für das lichtaktivierte Ätzen von Si- Wafern zum Einsatz kommt.

### 6.2.2 Fertige Träger - Synthese beim Hersteller

Beim Vertrieb fertiger Träger erfolgt die Synthese beim Hersteller. Dieser benötigt hierfür eine entsprechend leistungsfähige Syntheseeinheit, welche möglichst lange Oligos (3 oder mehr Basen lang) als Einsatzstoffe verwendet, die parallel in die Kanäle eingebracht (eingespritzt) werden, und so die Synthesezeiten pro Träger minimieren (Multiplexing). Hierbei ist es möglich, in den Trägern spezielle Zugänge vorzusehen, mit dem Ziel möglichst viele parallele und damit kurze Kanäle zu erhalten, unabhängig davon, welche Kanalstruktur für den Analysevorgang vorgesehen ist.

### 6.2.3 Einsatzstoffe im Träger

Für Anwendungen, wo es nicht auf eine schnelle Synthese der Träger, aber auf eine individuelle Gestaltung der Arrays ankommt, ist eine Bereitstellung der Einsatzstoffe (G, A, C, T und Puffer etc.) direkt im Träger in entsprechenden Reservoirs möglich. Die überflüssigen Einsatzstoffe müssen in einer entsprechenden Kammer im Träger aufgefangen werden. Das Volumen einer solchen Kammer ist durch eine Ausdehnung in der dritten Dimension nach oben oder unten problemlos auf ein Vielfaches des gesamten Kanalvolumens auslegbar: Eine Anwendungen dieser Träger-Variante ist gerade für Forschungslabors, aber auch kleine Arztpraxen denkbar.

Das Prinzip der Kapillarkraft kann hierbei in einer möglichen Ausführungsvariante direkt für den Fluidtransport im Träger verwendet werden. Jegliche Mechanik würde entfallen und die Befüllung der Kapillaren mit den Einsatzstoffen sowie der Probe könnte über die einfache Verstellung eines Ventils im Träger erfolgen. Die "Abfallkammer" könnte durch die Einbettung eines geeigneten Vlies-Stoffes eine unterstützende Saugwirkung entwickeln. Um eine Minimierung der benötigten Fluidmengen zu erreichen, sollte bei diesen Einwegströmungs- Ausführungen (keine Zirkulation und damit Wiederverwendung der Einsatzstoffe) auf immer gleich lange Kapillaren geachtet werden. Dies ist ebenfalls von Bedeutung für das Funktionieren der Kapillarkraft als Pumpe.

Ein weitere Variante ist eine vertikale Ausrichtung der planaren Träger, so dass auch Gravitationskräfte für den Fluidtransport im Träger genutzt werden können. Wenn diese Kräfte nicht ausreichen, um alle notwendige Fluidtransporte in den Träger zu realisieren, so sind andere geeignete Pumpmechanismen vorzusehen. Eine Möglichkeit hierzu ist eine elektrophoretische Bewegung der Fluide durch - in den Träger integrierte - Elektroden, oder durch eine Volumenreduktion in Kammern des Träger durch einen entsprechenden Krafteintrag von außen in den Träger (klassische Pumpe).

### 6.2.4 Einsatzstoffe in der Syntheseeinheit

Die Bereitstellung der Einsatzstoffe für die Träger-Synthese in Vorratsbehältern bietet prinzipiell den Vorteil des Multiplexens von fertigen Basen-Sequenzen und parallelen Kanälen, weshalb diese Ausführungsvariante für (Ultra)Hochdurchsatz-Screening und Träger-Hersteller empfehlenswert ist. Das Multiplexen kann an der Schnittstelle zum Träger erfolgen, in dem eine spezifische Basen- Sequenz für jeden Synthese-Zyklus einen anderen Kanal benetzt. Eine technisch aufwendigere, aber ggf. zuverlässigere Methode ist ein Multiplexen im Gerät durch ein entsprechendes Ventilsystem. Hier ist die Verschleppung zu beachten, welche durch die Verwendung unterschiedlicher Basen-Sequenzen entstehen kann.

Ein weiterer Punkt, welcher berücksichtigt werden muss, ist das Auffangen und Beseitigen des überschüssigen Materials am Ausgang der einzelnen Kanäle. Hier ist sowohl eine Zirkulation (Wiederverwendung des austretenden Materials) als auch eine Beseitigung der austretenden Einsatzstoffe denkbar.

### 6.3 Analytbestimmung

Die Analyse von Nukleinsäure- Sequenzen erfolgt wie bei anderen Oligonukleotid-Arrays durch Hybridisieren von Nukleinsäuren im Probenmaterial an komplementäre Stränge unter den immobilisierten Oligonukleotiden.

Als eine weitere mögliche Anwendung des Trägers können auch Peptidsequenzen in den Kanälen angekoppelt werden, ebenfalls nach in situ Syntheseprinzipien. Solche Peptide sind zu vielfältigen und teilweise hochspezifischen Bindungsreaktionen mit Peptiden, Proteinen und anderen Substanzen in der Lage, so dass sich das Spektrum potentieller Analyten erheblich ausweiten läßt.

Die Synthese im Träger würde erstmals massiv parallele und gleichzeitig kostengünstige Peptid-Arrays für eine Vielzahl von Anwendungen zur Verfügung stellen.

### 6.3.1 Analyten

Beispiele für Analyten sind Nukleinsäuren (DNA, RNA, in Spezialfällen auch PNA). Diese Nukleinsäuren können aus Gesamtgenomen, Fragmenten davon, Chromosomen, Plasmiden oder synthetischen Quellen (z.B. cDNA) gewonnen werden. In einer Ausführungsform kann das Probenmaterial aus dem menschlichen Genom stammen.

Weitere Beispiele für Analyten sind Proteine, Polypeptide und Peptide in allen Erscheinungsformen z.B. Hormone, Wachstumsfaktoren, Enzyme, Tumorantigene, Serumfaktoren, Antikörper, Carbohydrate, z.B. verschiedene Zucker in Lebensmitteln oder Agrarpflanzen, funktionelle Zucker, Polymere und andere organische Moleküle, z.B. drugs of abuse', Pharmaka, Metabolite, Aminosäuren, Transmitter, Pestizide, Insektizide, Lacke, verschiedene Toxine etc.

### 6.3.2 Varianten zur Bindung an den immobilisierten Interaktionspartner (Rezeptor)

Die Bindung des Analyten an den Rezeptor kann bei Nukleinsäuren durch Hybridisierung von komplementären Nukleinsäuren z.B. längere Moleküle wie cDNA, synthetische Oligonukleotide, PNA, RNA erfolgen. Peptide als Rezeptoren , z.B. synthetische Peptide oder natürliche Peptide können über Protein-Protein oder Protein-Nukleinsäure-Wechselwirkungen an den Analyten binden.

### 6.3.3 Varianten zur Signalerzeugung

Zwei Prinzipien zur Signalerzeugung werden bevorzugt eingesetz, nämlich: die direkte Detektion eines vorher oder in der Reaktion markierten Analyten (bevorzugte Methode in der Nukleinsäureanalytik mittels Hybridisierung) und die indirekte Detektion durch Kompetition des Analyten bzw. der Zielsequenz mit einem markierten Standard. Die erste Variante ist für einige Anwendungen gut etabliert, für Diagnostik z.B. von Serumkomponenten aber eher schlecht geeignet, die mit Peptid-Arrays auch im Träger möglich ist. Die zweite Variante ist für diese Anwendungen daher vorzuziehen, außerdem erlaubt sie prinzipiell eine einfachere Probenvorbereitung durch den Anwender.

Eine direkte Detektion kann erfolgen durch Markierung der Analyten mit einem Farbstoff für die Absorptionsmessung, einem Fluoreszenzfarbstoff, Markierung der Analyten mit Reporterenzym, anschließend Reaktion (z.B. Chemo- oder Biolumineszenz), selektive Markierung des gebundenen Analyten, z.B. bei Nukleinsäuren durch interkalierende (Fluoreszenz-) Farbstoffe, Doppelstrang-bindende Proteine oder Doppelstrang-bindende Antikörper oder eine sekundäre Detektion des gebundenen Analyten mit einer zweiten Komponente, z.B. bei PNA-DNA Hybriden durch DNA spezifischen Antikörper. Als markierte Standards verwendet werden können enzymgekoppelte Standards (z.B. Chemo- und Biolumineszenz mit alkalischer Phosphatase, Peroxidase etc.) oder Fluoreszenz-) Farbstoff gekoppelte Standards. Proteinstandards können als Fusionsproteine mit einem Reporterenzym (siehe oben) oder einem autofluoreszierendem Protein (z.B. GFP), z.B. für rekombinante Antikörper, Protein-Hormone, Wachstumsfaktoren etc. eingesetzt werden.

### 6.4 Bereitstellung des Probenmaterials

Für die Bereitstellung des Probenmaterials gibt es ebenfalls wieder unterschiedliche Ausführungsvarianten. Für die eigentliche Detektion ist die Art der Bereitstellung nicht relevant, da an der Schnittstelle immer in Flüssigkeit gelöste DNA-Fragmente in ausreichender Menge für die angestrebte Untersuchung bereitzustellen sind.

### 6.4.1 Externe Probenvorbereitung

Die Probenvorbereitung kann entweder manuell im Labor, in einem getrennten Analysesystem oder in einer in das gleiche System integrierten Vorbereitungseinheit erfolgen. Die detektionsbereite Probe wird dann mittels manuellem oder automatischem Pipettieren oder vergleichbaren Verfahren in den Träger eingebracht.

### 6.4.2 Probenvorbereitung im gleichen Träger "all in one"

Gerade, wenn das Multiplexen bei der Träger-Synthese zur Reduktion der Produktionszeiten verwendet wird, können gleiche oder sogar kürzere Zeiten für die Rezeptorsynthese erreicht werden, als z.B. für die DNA-Amplifizierung der Probe mittels PCR notwendig wäre. Dadurch wird eine Integration einer PCR in das Synthese- System oder sogar in den Träger für viele Anwendungen sinnvoll.

Neben der zeitintensiven PCR ist auch der vorgelagerte Zellaufschluss zum Beispiel über gut automatisierbare Verfahren wie Ultraschall oder Hochspannung ebenso wie die DNA-Isolierung integrierbar.

### 6.5 Detektionseinheit

Die Auslesung der Lichtsignale für die Nachweisreaktionen im Träger-Array soll in einer Detektionseinheit erfolgen, wobei die Anregungslichtquelle (Fluoreszenz, Lumineszenz oder Absorption als optischer Nachweis) dem CCD-Chip zur Lichtsignalmessung direkt gegenüber angeordnet wird. Der Träger-Array befindet sich zwischen Lichtquelle und Detektions-Chip (Sandwichbauweise). Als Anregungs-lichtquelle kann eine Belichtungsmatrix herangezogen werden. Die räumliche Anordnung dieser Einheit kann je nach Bedarf erfolgen (z.B. Nutzung der Gravitation für Strömungsvorgänge im Chip). Durch diese möglichst kompakte Bauweise werden die Lichtlaufwege und damit auch die benötigte Lichtintensität minimiert. Auf die Verwendung einer aufwendigen, platzintensiven, lichtschluckenden und teuren Optik soll sowohl auf der Anregungs-, als auch auf der Detektionsseite verzichtet werden.

### 6.5.1 Temperatur bei der Hybridisierung

Die Temperierung (derzeit typischerweise bei 60°C - neueste Entwicklungen ermöglichen auch schon eine Hybridisierung bei 25°C mit Niedrigsalz- Bedingungen) bei der Hybridisierung kann entweder durch entsprechende Temperaturelemente in der Detektionseinheit oder durch die Anregungslichtquelle bzw. das Anregungslicht an sich erfolgen. Temperaturelemente in den Trägern sind ebenfalls möglich.

### 6.5.2 Anregungslichtquelle

Als Lichtquellen kommen je nach den Markern der Analyten (Nachweisverfahren via Absorption oder Fluoreszenz etc.) hochparalleles Licht aus einer Lampe (Weißes Licht), hochparalleles Licht aus einer Blitzröhre, hochparalleles monochromatisches Licht, ein monochromatischer Laserlichtstrich, eine flächige Belichtung mittels Aufweitung des Laserstrahls, ein monochromatischer Laserstrahl oder eine programmierbare Lichtquellenmatrix in Frage.

Gegebenenfalls kann ein entsprechendes optisches Gitter oder eine entsprechende Optik zwischen Anregungslichtquelle und Träger-Array vorgesehen sein.

### 6.5.3 CCD-Kamera Detektion

Die Detektionseinheit besteht vorzugsweise nur aus einem CCD-Chip. Diese haben aktuell auf einer Fläche von beispielsweise 25 x 37 mm etwa 2000 x 3000 Pixel (Cannon). Ordnet man auf einer solchen Fläche von 25 x 37 mm etwa 500 parallele Kanälen mit ca. 20 *µ*m Durchmesser an (jede zweite Doppel-Pixelreihe), so erhält man in jedem Kanal 750 Messpunkte (Felder), wenn man nur jeden zweiten Doppel-Pixel unter dem Kanal nutzt. Damit hätte man 375.000 Reaktionsbereiche auf einem einzigen Träger, wobei jeder Reaktionsbereich 4 Farb- bzw. 12 Schwarzweiß-Pixel überdeckt und eine Fläche von 20 x 20 *µ*m hat. Die Lichtsignale müssen möglichst dicht am optischen CCD-Chip erzeugt werden, damit eine fehlerhafte Zuordnung von Lichtsignalen und Messpunkten mit ihrer spezifischen Basen- Sequenz sowie eine Überlagerung benachbarter Lichtsignale ausgeschlossen werden kann. Ansonsten kann eine serielle Detektion sich überlagernder Bereiche erfolgen oder es werden faseroptische Elemente eingesetzt.

Die entstehende Vielzahl an Messwerten (4 x 500 x 750 = 1.5 Mio. Farbsignale bzw. 4.5 Mio. Intensitätswerte zwischen 0 und 4096 Digitalwerten), welche zur Verfügung stehen (aktueller Stand der CCD-Chip Technik), bilden die Basis welche eine umfangreiche Statistik bei der Analyse der detektierten Lichtsignale erlaubt. Das Bearbeiten der anfallenden Datenmengen ist, durch die Entwicklung der Leistungsfähigkeit bei gleichzeitigem Preisverfall von modernen Rechnersystemen, problemlos möglich.

Die Detektion der Nachweisreaktion kann sowohl qualitative als auch quantitative Aussagen machen und zwar welche Fängermoleküle (Position im Array) haben Anlagerungspartner gefunden (Auswertung der z.B. fluoreszenzmarkierten Labels) und wieviele Fängermoleküle einer Klasse haben einen Hybridisierungspartner gefunden.

Gegebenenfalls kann ein entsprechendes optisches Gitter oder eine entsprechende Optik zwischen dem Träger-Array und dem CCD-Kamera Chip vorgesehen sein.

Wenn die Detektion mit einer CCD-Kamera bzw. einem CCD-Chip keine ausreichenden Signale ergibt, kann die Detektion im Analysesystem auch mittels anderer, empfindlicherer Sensoren erfolgen.

Interessant im Zusammenhang mit der vorliegenden Erfindung ist die Verwendung einer Inspektionseinheit, wie in der deutschen Patentanmeldung 198 39254.0 beschrieben ist. Diese Inspektionseinheit umfasst eine elektronisch steuerbare Lichtquellenmatrix und eine der Lichtquellenmatrix zugewandt-gegenüberliegende Lichtsensormatrix, nämlich CCD-Bildaufnehmer.

In diesem Zusammenhang ist es denkbar, dass der Anwender sich seine Träger selbst erzeugt und direkt verwendet. Er lädt sich einfach die benötigten Daten (DNA-Sequenzen) von einer CD-ROM oder aus dem Internet und erzeugt in seiner Belichtungsmatrix-CCD-Einheit seinen individuellen DNA-Chip, benetzt ihn anschließend mit der Probe und liest die Signale aus.

Misst man z.B. jeden zweiten Pixel in dieser Anordnung für die Photoaktivierung, so kann man die Pixel dazwischen, welche in Projektion innerhalb eines Kanals liegen, für eine permanente Prozesskontrolle verwenden. So kann man z.B. das Einströmen einer Gasblase zwischen zwei Fluiden in einem Kanal individuell und dynamisch verfolgen. Auch ein Färben der Trägerfluide für G, A, C und T wäre denkbar, so dass die Anwesenheit der richtigen Oligos überprüfbar würde und eine Farbveränderung könnte eine Verschleppung signalisieren. Bei der anschließenden Detektion könnte wiederum eine ortsspezifische und wenn notwendig sogar farbspezifische Lichtanregung erfolgen. Hierdurch ergeben sich ganz neue Möglichkeiten für Nachweisverfahren, wie sie derzeit noch nicht vorhanden sind.

Durch die Inspektionseinheit (Belichtungsmatrix-CCD-Einheit) können die Strömungsvorgänge in den Kanälen in einem Träger sowohl während der Produktion - sprich der Oligo-Synthese - als auch während der Analyse überwacht werden. Hierzu können z.B. Reinigungsgasblasen zwischen zwei Fluiden in den Kanälen oder eine Färbung der einzelnen Fluide verwendet werden.

Für die lichtinduzierte Abspaltung von Schutzgruppen während der Synthese von DNA-Oligos auf oder in dem Träger kann eine Belichtungsmatrix dienen, die die notwendige Wellenlänge von z.B. 360-370 nm erzeugt und überträgt.

Die Detektion der Nachweisreaktion im Träger kann ebenfalls in der Inspektionseinheit erfolgen. Wenn der Nachweis über Fluoreszenzmarker realisiert wird, müsste hierzu ggf. die Hintergrundbeleuchtung gewechselt werden (automatisch möglich), wobei optische Filter oder/und Glasfaserelemente ("Taper") verwendet werden können. Gegebenenfalls kommen hier auch neue Detektionsverfahren zum Einsatz, welche erst durch die extrem flexible, individuelle Anstrahlung und Detektion des einzelnen Reaktionsbreichs möglich werden.

Für eine Standard-Hybridisierung von DNA-, RNA- und PNA-Strängen miteinander benötigt man eine Temperatur von ca. 55 - 65°C. Im einfachsten Fall kann diese Temperatur durch die abgestrahlte Energie der Belichtungsmatrix erzeugt werden (Abwärme und Wellenlänge). Damit ließe sich eine weitere Kompaktierung der Anordnung erreichen.

### 8. Exemplarische Ausführungsformen

Die Synthese von DNA-Molekülen in Kanälen kann unter Verwendung von Standard-Synthonen, z.B. Phosphoramidit-Bausteinen, mit geeigneten Schutzgruppen, z.B. Dimethoxymethyl (DMT) erfolgen. Ausgehend von einem an die Festphase gekoppelten Linker kann eine entsprechende fluidische DNA-Synthese erfolgen.

Dieses Format kann für die bevorzugte Ausführungsform der Erfindung mit einer lichtabhängigen Steuerung der DNA-Synthese kombiniert werden. Dazu sind Schutzgruppen bekannt, die eine lichtabhängige Entschützung erlauben, so dass die Schutzgruppe, die meist am 5'-Kohlenstoffatom des Synthons gebunden ist, durch Licht geeigneter Wellenlänge abgespalten wird. Auf diese Weise ist in Kapillaren die Synthese von Nukleinsäuren mit einer Länge von 18 oder mehr Nukleotiden möglich.

Durch Ablösung des synthetisierten DNA-Oligomere, wie es durch Verwendung geeigneter Linker möglich ist, können die Reaktionsprodukte z.B. durch Hochleistungflüssigchromatographie (HPLC) analysiert werden. Dabei lässt sich über den Anteil der Vollängenprodukte die Effizienz der kapillaren DNA-Synthese zeigen.

Für die lichtabhängige DNA-Synthese wird der Reaktionsbereich auf dem Träger orts- oder/und zeitspezifisch mit einer geeigneten Lichtquelle belichtet, z.B. mit einer Quecksilberdampflampe, Laserlicht (z.B. Stickstofflaser mit 373 nm) oder mit einer UV-LED. Ebenso geeignet sind auch andere Lichtquellen, die eine ausreichend energiereiche Strahlung aufweisen.
- Fig. 1: zeigt in einer stark schematisierten Draufsicht einen Träger nach der Erfindung.
- Fig. 2: zeigt Beispiele für Kanalanordnungen in einem Träger nach der Erfindung.
- Fig. 3: zeigt eine schematische Darstellung eines Trägers in einer Inspektionseinheit aus programmierbarer Lichtquellenmatrix und CCD-Matrix.
- Fig. 4: zeigt eine schematische Darstellung einer erfindungsgemäßen Vorrichtung für ein lichtgestütztes integriertes Synthese- und Analyseverfahren und
- Fig. 5: zeigt den Aufbau aus Fig. 4 für eine fluidische Individualisierung von Reaktionsbereichen.

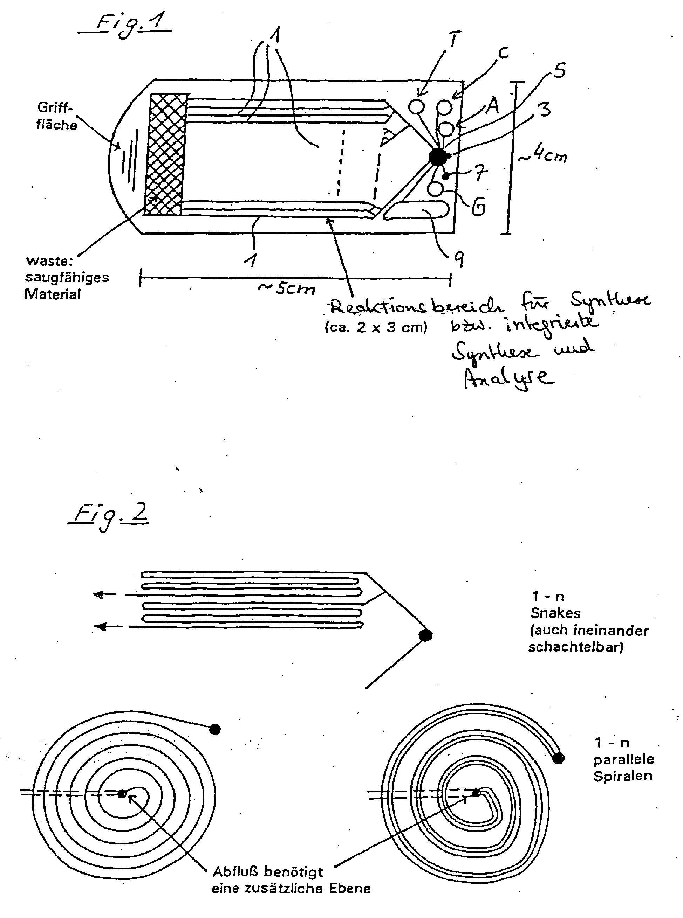

Figur 1 zeigt einen den transparenten Träger in einer Draufsicht in stark schematisierter Weise. Man erkennt die parallel zueinander verlaufenden Kanäle 1, beispielsweise 500 Kanäle mit einer Länge von 37 nm. Mit T, G, A, C sind in Fig. 1 Reservoirs für die einzelnen Einsatzstoffe (Basen) bezeichnet. Mit 3 ist der Gaseinlass bezeichnet. 5 kennzeichnet ein Ventil. 7 kennzeichnet die Probeneingabe und mit 9 ist ein Zugang für weitere Synthesechemikalien sowie Reinigungs-/Waschflüssigkeit bezeichnet.

In Figur 2 sind schematisch weitere Beispiele für alternative Kanalanordnungen dargestellt.

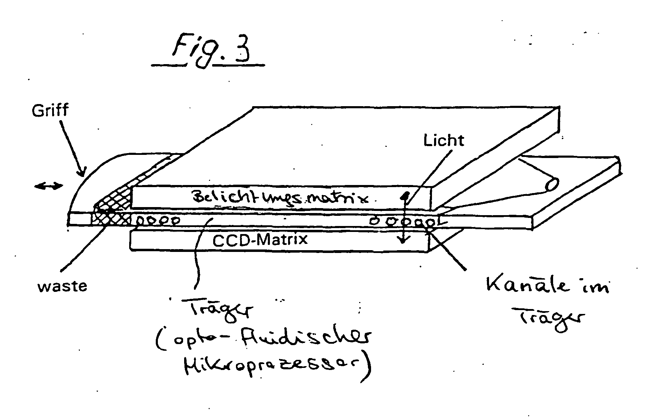

Figur 3 zeigt den Träger nach Fig. 1 in einer Inspektionseinheit aus programmierbarer Lichtquellenmatrix, z.B. einer LCD-Matrix und einer CCD-Detektionsmatrix.

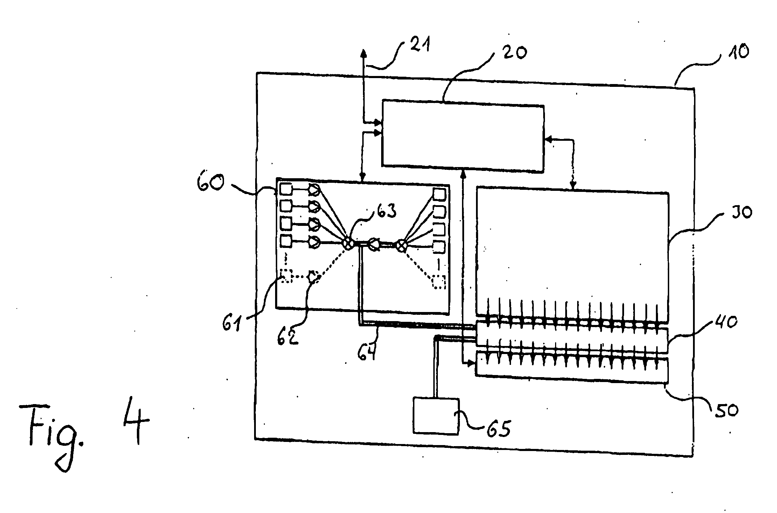

In Figur 4 ist eine erfindungsgemäße Vorrichtung mit einem auswechselbaren Träger 40 dargestellt, wobei der prinzipielle Aufbau unabhängig davon ist, ob der Träger in jedem Zyklus oder erst bei Verschleiß ausgewechselt wird. In letzterem Fall findet eine Reinigung und anschließende Wiederverwendung derselben Kanäle statt. Dargestellt ist eine programmierbare Lichtquellenmatrix 30. Deren Programmierbarkeit kann in die Systemkomponente 20, die aus einem Rechner bzw. einem Computer besteht, integriert sein, so dass nur eine frei ansteuerbare Lichtquellenmatrix als Komponente 30 notwendig ist. Diese Lichtquellenmatrix 30 strahlt Licht definierter Wellenlänge und Intensität auf beliebig ansteuerbare Orte einer zumindest zweidimensionalen Matrix, die der hochparallelen Belichtung der Reaktionsbereiche im Träger 40 dient. Besagter Träger 40 wird durch die Lichtquellenmatrix 30 mit dem rechnergesteuerten Lichtmuster bestehend aus Energiewellen in allen Reaktionsbreichen individuell bestrahlt. Über das fluidische Anschlusssystem 64 werden die vom Fluidikmodul 60 bereitgestellten Fluide in den Träger 40 transportiert und in dessen in der Zeichnung nicht dargestellten Mikrostruktur in geeigneter Weise an die Reaktionsbereiche weitergeleitet. Dadurch wird der Träger 40 zu einem opto-fluidischen Mikroporzessor. Dieser kann entweder nach jeder Anwendung gewechselt werden oder nach jeder Anwendung gereinigt und nur zu Servicezwecken bei Verschleiß gewechselt werden.
Das eintretende Licht kann zum Beispiel für Absorptionsmessungen, die Aktivierung von Photoreaktionen oder das Anregen von Fluoreszenz genutzt werden.

Das aus dem Träger 40, bzw. dem opto-fluidischen Mikroprozessor austretende Licht kann beispielsweise das im Durchlicht den Träger passierende Licht der Lichtquellenmatrix 30 sein. Es kann sich dabei jedoch auch um Lichtsignale handeln, die in den einzelnen Reaktionsbereichen des Trägers 40 durch beispielsweise Fluoreszenz oder Lumineszenz erzeugt werden.

Die Detektormatrix 50, die zum Beispiel aus einem CCD-Chip mit oder ohne Optik besteht, ist so gegenüber einer Lichtquellenmatrix 30 mit einem dazwischen liegenden Träger 40 angeordnet, dass dadurch eine dreifache Matrixanordnung aus Licht-, Träger und Detektormatrix entsteht.

Das Fluidmodul 60 dient der Versorgung des Reaktionsträgers 40 zum Beispiel mit Einsatzstoffen, Schutzgasen, Chemikalien, wie Lösemitteln, etc. und Probenmaterial. Das Fluidmodul 60 besteht aus Tanks 61, die durch Pumpen 62 und Ventile 63 in geeigneter Weise entleert werden. Die Tanks können einzeln oder im Cluster ausgewechselt oder neu gefüllt werden. Permanent benötigte Fluide, wie beispielsweise Schutzgas, können auch mittels Leitungen kontinuierlich (ohe Tanks im System) zugeführt werden. Der fluidische Abfall der verschiedenen Verfahren kann entweder im Träger 40 in integrierten Tanks oder in einem Abfallsystem 65 oder bei Clustern außerhalb des einzelnen Systems aufgefangen werden.

Ebenfalls dargestellt ist die Systemgrenze 10 der Vorrichtung, die als Einzelgerät oder auch in zentralen oder dezentralen Clustern eingesetzt werden kann. Diese Cluster sind immer informationstechnisch miteinander verknüpft. Die an einem Ort befindlichen Systeme können auch gemeinsam durch manuelle Bedienung oder automatisierte Komponenten mit Energie, Fluiden, wie Einsatzstoffen, Reaktionschemikalien, Schutzgasen und Probenmaterial, sowie mit den benötigen Trägern versorgt werden.

Die Systemkomponente 20 in Form eines Computers oder Rechners übernimmt die Steuerung bzw. Regelung des Systems. Hierunter fallen auf der Basis der Berechnung der Sonden- bzw. Rezeptorsequenzen für die einzelnen Reaktionsbereiche die Steuerung der Lichtquellenmatrix 30 sowie der Fluidkompnente 60. Ferner werden die Daten der Detektormatrix 50 erfasst und ausgewertet.

Jede Vorrichtung kann somit über seine Systemgrenze 10 hinweg mit anderen Vorrichtungen oder Systemen, bestehend aus wiederum einer erfindungsgemäßen Vorrichtung oder anderen Rechnern oder Datenbanken, kommunizieren. Dies kann beispielsweise über Leitungen, Bussysteme oder über das Internet erfolgen. Dabei kann die Kommunikation zentral koordiniert über Leitrechner erfolgen oder als Cluster gleichberechtigter Systeme. Ebenfalls vorgesehen ist eine Datenschnittstelle 21 zur Systemumgebung.

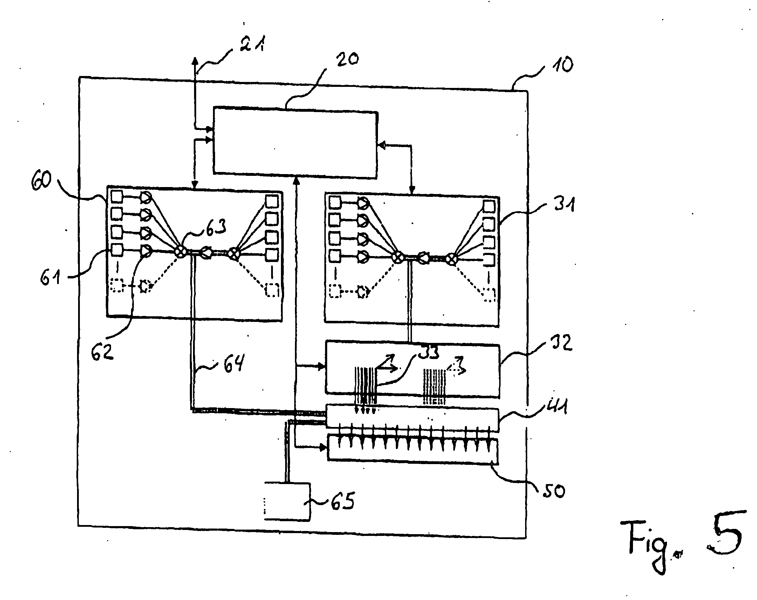

Figur 5 zeigt den Aufbau aus Figur 4 für eine fluidische Individualisierung der Reaktionsbereiche. Wiederum dargestellt ist ein Träger 41. Dieser wird durch das fluidische Entschützungsmodul 32 rechnergesteuert individuell benutzt. Über das fluidische Anschlusssystem 64 werden die vom Fluidmodul 60 bereitgestellten Fluide in den Träger transportiert und in dessen in der Zeichnung nicht dargestellten Mikrostruktur in geeigneter Weise an die Reaktionsbereiche weitergeleitet. Dadurch wird der Träger 41 zu einem opto-fluidischen Mikroprozessor. Dieser kann entweder nach jeder Anwendung gewechselt werden oder nach jeder Anwendung gereinigt und nur zu Servicezwecken bei Verschleiß gewechselt werden.

In diesen Träger kann zum Beispiel von oben oder/und von der Seite Licht für die Anregung von Fluoreszenzreaktionen etc. eingespeist werden.

Das aus dem Träger, bzw. dem opto-fluidischen Mikroprozessor austretende Licht kann beispielsweise durch Lumineszenz an den Reaktionsbereichen erzeugt werden.

Das fluidische Entschützungsmodul32 kann jeden Reaktionsbereich auf dem Träger 41 mit mindestens einer der Benetzungskomponenten 33 (z.B. Düsen, Kapillaren, etc.) individuell mit Fluiden in Kontakt bringen. Hierdurch können zum Beispiel lokal chemische und biochemische Reaktionen aktiviert werden.

Das Fluidmodul 31 dient der Versorgung des fluidischen Entschützungsmoduls 32 mit Einsatzstoffen oder Chemikalien. Das Fluidmodul 31 ist dem Modul 60 vergleichbar aufgebaut und besteht je nach Bedarf aus Tanks, Leitungen, Ventilen, etc.

Die Detektormatrix 50, die zum Beispiel aus einem CCD-Chip mit oder ohne Optik besteht, ist so gegenüber einem fluidischen Entschützungsmodul 32 mit einem dazwischen liegenden Träger 41 angeordnet, dass dadurch wiederum eine dreifache Matrixanordnung entsteht.

Das Fluidmodul 60 dient der Versorgung des Trägers 41 zum Beispiel mit Einsatzstoffen, Schutzgasen, Chemikalien, wie Lösemitteln, etc. und Probenmaterial. Das Fluidmodul 60 besteht aus Tanks 61, die durch Pumpen 62 und Ventile 63 in geeigneter Weise entleert werden. Die Tanks können einzeln oder im Cluster ausgewechselt oder neu gefüllt werden. Permanent benötigte Fluide, wie beispielsweise Schutzgas, können auch mittels Leitungen kontinuierlich (ohne Tanks im Ssystem) zugeführt werden. Der fluidische Abfall der verschiedenen Verfahren kann entweder im Träger 41 in integrierten Tanks oder in einem Abfallsystem 65 oder bei Clustern außerhalb des einzelnen Systems aufgefangen werden.

Wiederum dargestellt ist die bereits erläuterte Systemgrenze 10 der Vorrichtung und die Systemkomponente 20 in Form eines Computers oder Rechners, der die Steuerung bzw. Regelung des System übernimmt. Hierunter fallen auf der Basis der Berechnung der Sondensequenzen für die einzelnen Reaktionsbereiche die Steuerung der Fluidmodule 31 und 60, sowie des fluidischen Entschützungsmoduls 32. Ferner werden die Daten der Detektormatrix 50 erfasst und ausgewertet.

Jede Vorrichtung kann somit über seine Systemgrenze 10 hinweg mit anderen Vorrichtungen oder Systemen, bestehend aus wiederum einer erfindungsgemäßen Vorrichtung oder anderen Rechnern oder Datenbanken, kommunizieren. Dies kann beispielsweise über Leitungen, Bussysteme oder über das Internet erfolgen. Dabei kann die Kommunikation zentral koordiniert über Leitrechner erfolgen ode als Cluster gleichberechtigter Systeme. Ebenfalls vorgesehen ist eine Datenschnittstelle 21 zur Systemumgebung.

## Patentansprüche

1. Verfahren zur Herstellung eines Trägers für die Bestimmung eines Analyten, umfassend die Schritte
(a) Bereitstellen eines unmarkierten Trägerkörpers,
(b) Einbringen des Trägerkörpers in eine Syntheseeinheit,
(c) Entnehmen von Informationen über Sequenzen von auf dem Träger zu synthetisierende Rezeptoren aus einer Datenbank,
(d) Leiten einer Flüssigkeit mit darin enthaltenen Rezeptorbausteinen über den Trägerkörper,
(e) orts- oder/und zeitspezifisches Immobilisieren der Rezeptorbausteine an jeweils vorbestimmten Positionen auf dem Trägerkörper durch Belichten über eine programmierbare Lichtquellenmatrix, und
(f) Wiederholen der Schritte (d) bis (e), bis die gewünschten Rezeptoren an den jeweils vorbestimmten Positionen synthetisiert worden sind.

2. Verfahren nach Anspruch 1,
**dadurch gekennzeichnet,**
**dass** der unmarkierte Trägerkörper gemäß (a) von einem Hersteller bereitgestellt und an einen Kunden als Besitzer der Syntheseeinheit gemäß (b) geliefert wird.

3. Verfahren nach Anspruch 1 oder 2,
**dadurch gekennzeichnet,**
**dass** gemäß Schritt (c) die Sequenzen aus einer frei zugänglichen Datenbank entnommen werden.

4. Verfahren nach Anspruch 1 oder 2,
**dadurch gekennzeichnet,**
**dass** gemäß Schritt (c) die Sequenzen aus einer vom Hersteller des unmarkierten Trägerkörpers gemäß (a) bereitgestellten Datenbank entnommen werden.

5. Verfahren nach einem der Ansprüche 1 bis 4,
**dadurch gekennzeichnet,**
**dass** man den Träger zur Bestimmung von Analyten in der Probe verwendet:

6. Verfahren nach Anspruch 5,
**dadurch gekennzeichnet,**
**dass** mehrere Synthese/Analytbestimmungszyklen durchgeführt werden, wobei die Rezeptoren für einen nachfolgenden Zyklus auf Basis der Informationen aus einem vorhergehenden Zyklus synthetisiert werden.

7. Verfahren nach Anspruch 5 oder 6,
**dadurch gekennzeichnet,**
**dass** man eine aus einem Patienten stammende Probe bestimmt.

8. Verfahren nach Anspruch 7,
**dadurch gekennzeichnet,**
**dass** man die Ergebnisse der Bestimmung in den individuellen Unterschieden des Patienten abgleicht und aus den Ergebnissen einen zweiten Träger bildet, welcher eine Bestimmung, angepasst an den individuellen Patienten durchführt.

9. Verfahren nach einem der vorhergehenden Ansprüche,
**dadurch gekennzeichnet,**
**dass** man den Trägerkörper in eine integrierte Synthese- und Analyseeinheit einbringt, den Träger nach der Synthese der Rezeptoren mit einer Analyten enthaltenden Probe in Kontakt bringt und die Analyten über deren Bindung an die auf dem Träger immobilisierten Rezeptoren bestimmt.

10. Verfahren nach einem der vorhergehenden Ansprüche,
**dadurch gekennzeichnet,**
**dass** der Trägerkörper mindestens einen Kanal umfasst.

11. Verfahren nach einem der vorhergehenden Ansprüche,
**dadurch gekennzeichnet,**
**dass** der Trägerkörper eine Vielzahl von Kanälen enthält, die vorzugsweise parallel zueinander angeordnet sind.

12. Verfahren nach einem der vorhergehenden Ansprüche,
**dadurch gekennzeichnet,**
**dass** die Rezeptoren aus Nukleinsäuren und Nukleinsäureanaloga ausgewählt werden.

13. Verfahren nach Anspruch 12,
**dadurch gekennzeichnet,**
**dass** die Rezeptorbausteine aus Nukleotiden, Oligonukleotiden, Nukleotidanaloga und Oligonukleotidanaloga ausgewählt werden.

14. Verfahren nach einem der Ansprüche 1 bis 11,
**dadurch gekennzeichnet,**
**dass** die Rezeptoren aus Polypeptiden ausgewählt werden.

15. Verfahren nach Anspruch 14,
**dadurch gekennzeichnet,**
**dass** die Rezeptorbausteine aus Aminosäuren und Peptiden ausgewählt werden.

16. Verfahren nach einem der vorhergehenden Ansprüche,
**dadurch gekennzeichnet,**
**dass** ein Reagenzienkit, umfassend einen Träger und Bausteine für die Synthese polymerer Rezeptoren auf dem Träger bereitgestellt wird.

17. Verfahren nach einem der vorhergehenden Ansprüche,
**dadurch gekennzeichnet,**
**dass** der Trägerkörper in eine Syntheseeinheit eingebracht wird, umfassend eine programmierbare Lichtquellenmatrix, eine Detektormatrix, Mittel zur Zufuhr von Fluids in den Träger und zur Ableitung von Fluids aus dem Träger, sowie elektronische Steuerungs- und Regelungsmittel, wobei der Trägerkörper zwischen der programmierbaren Lichtquellenmatrix und der Detektormatrix angeordnet wird.

18. Verfahren nach einem der vorhergehenden Ansprüche,
**dadurch gekennzeichnet,**
**dass** die Syntheseeinheit weiterhin Mittel zur Entschützung von Reaktionskomponenten auf dem Träger umfasst.

19. Verwendung des Verfahrens nach einem der Ansprüche 1 bis 18 zur Sequenzierung von Nukleinsäuren.

20. Verwendung nach Anspruch 19 zur Neusequenzierung oder/und Resequenzierung von komplexen genetischem Material, wie etwa individueller Genome oder synthetischen Nukleinsäuren.

21. Verwendung des Verfahrens nach einem der Ansprüche 1 bis 18 zur Gewinnung diagnostischer Informationen für die individuelle Patientenversorgung, wie etwa die individuelle Wirkung von Pharmaka.

22. Verwendung des Verfahrens nach einem der Ansprüche 1 bis 18 zur Wirkungsanalyse pharmakologischer Substanzen.

23. Verwendung des Verfahrens nach einem der Ansprüche 1 bis 18 zur Erstellung und Analyse von Substanzbibliotheken.

24. Verwendung des Verfahrens nach einem der Ansprüche 1 bis 18 zum Vergleich von Individuen in einer Population.

25. System zur integrierten Synthese von Rezeptoren auf einem Träger, umfassend:
(a) einen Trägerkörper,
(b) eine Syntheseeinheit, umfassend eine programmierbare Lichtquellenmatrix,
(c) Mittel zur Synthese von Rezeptoren durch stufenweisen Aufbau aus Rezeptorbausteinen an jeweils vorbestimmten Positionen des Träger und
(d) eine Datenbank zum Entnehmen von Informationen über Sequenzen von auf dem Träger zu synthetisierenden Rezeptoren.

26. System nach Anspruch 25,
**dadurch gekennzeichnet,**
**dass** der Trägerkörper mindestens einen Kanal umfasst.

27. System nach Anspruch 25 oder 26,
**dadurch gekennzeichnet,**
**dass** die Syntheseeinheit weiterhin eine Detektormatrix, Mittel zur Zufuhr von Fluids in den Träger und zur Ableitung von Fluids aus dem Träger, sowie elektronische Steuerungs- und Regelungsmittel umfasst.

28. System nach Anspruch 27,
**dadurch gekennzeichnet,**
**dass** der Trägerkörper zwischen der programmierbaren Lichtquellenmatrix und der Detektormatrix angeordnet ist.

## Claims

1. Method for producing a support for determining an analyte, comprising the steps of
(a) providing an unlabelled support body,
(b) introducing the support body into a synthesis unit,
(c) gathering information about sequences of receptors to be synthesized on the support from a database,
(d) passing a liquid with receptor building blocks contained therein over the support body,
(e) site- or/and time-specifically immobilizing the receptor building blocks in each case on predetermined positions on the support body by illumination via a programmable light source matrix, and
(f) repeating steps (d) to (e) until the required receptors have been synthesized in each case on the predetermined positions.

2. Method according to Claim 1, **characterized in that** the unlabelled support body specified in (a) is provided by a manufacturer and supplied to a customer as possessor of the synthesis unit specified in (b).

3. Method according to Claim 1 or 2, **characterized in that** the sequences are gathered in step (c) from a freely accessible database.

4. Method according to Claim 1 or 2, **characterized in that** the sequences are gathered in step (c) from a database provided by the manufacturer of the unlabelled support body specified in (a).

5. Method according to any of Claims 1 to 4, **characterized in that** the support is used to determine analytes in the sample:

6. Method according to Claim 5, **characterized in that** a plurality of synthesis/analyte determination cycles are carried out, with the receptors for a subsequent cycle being synthesized on the basis of the information from a preceding cycle.

7. Method according to Claim 5 or 6, **characterized in that** a sample originating from a patient is determined.

8. Method according to Claim 7, **characterized in that** the results of the determination are matched in the individual differences of the patient, and a second support which carries out a determination adapted to the individual patient is formed from the results.

9. Method according to any of the preceding claims, **characterized in that** the support body is introduced into an integrated synthesis and analysis unit, the support is brought into contact after the synthesis of the receptors with an analyte-containing sample, and the analytes are determined via their binding to the receptors immobilized on the support.

10. Method according to any of the preceding claims, **characterized in that** the support body includes at least one channel.

11. Method according to any of the preceding claims, **characterized in that** the support body comprises a large number of channels which are preferably arranged parallel to one another.

12. Method according to any of the preceding claims, **characterized in that** the receptors are selected from nucleic acids and nucleic acid analogues.

13. Method according to Claim 12, **characterized in that** the receptor building blocks are selected from nucleotides, oligonucleotides, nucleotide analogues and oligonucleotide analogues.

14. Method according to any of Claims 1 to 11, **characterized in that** the receptors are selected from polypeptides.

15. Method according to Claim 14, **characterized in that** the receptor building blocks are selected from amino acids and peptides.

16. Method according to any of the preceding claims, **characterized in that** a reagent kit including a support and building blocks for the synthesis of polymeric receptors is provided on the support.

17. Method according to any of the preceding claims, **characterized in that** the support body is introduced into a synthesis unit including a programmable light source matrix, a detector matrix, means for supplying fluids into the support and for discharging fluids from the support, and electronic control means, with the support body being disposed between the programmable light source matrix and the detector matrix.

18. Method according to any of the preceding claims, **characterized in that** the synthesis unit further includes means for deprotecting reaction components on the support.

19. Use of the method according to any of Claims 1 to 18 for sequencing nucleic acids.

20. Use according to Claim 19 for the new sequencing or/and resequencing of complex genetic material such as, for example, individual genomes or synthetic nucleic acids.

21. Use of the method according to any of Claims 1 to 18 for obtaining diagnostic information for individual patient management such as, for example, the individual effect of pharmaceuticals.

22. Use of the method according to any of Claims 1 to 18 for analysing the effect of pharmacological substances.

23. Use of the method according to any of Claims 1 to 18 for setting up and analysing substance libraries.

24. Use of the method according to any of Claims 1 to 18 for comparing individuals in a population.

25. System for the integrated synthesis of receptors on a support, including:
(a) a support body,
(b) a synthesis unit including a programmable light source matrix,
(c) means for synthesizing receptors by stepwise assembly from receptor building blocks in each case on predetermined positions of the support and
(d) a database for gathering information about sequences of receptors to be synthesized on the support.

26. System according to Claim 25, **characterized in that** the support body includes at least one channel.

27. System according to Claim 25 or 26, **characterized in that** the synthesis unit further includes a detector matrix, means for supplying fluids into the support and for discharging fluids from the support, and electronic control means.

28. System according to Claim 27, **characterized in that** the support body is disposed between the programmable light source matrix and the detector matrix.

## Revendications

1. Procédé de fabrication d'un support pour le dosage d'un analyte, comprenant les étapes consistant à :
(a) préparer un corps de support non marqué,
(b) introduire le corps de support dans une unité de synthèse,
(c) extraire des informations sur les séquences des récepteurs à synthétiser sur le support à partir d'une base de données,
(d) diriger un liquide avec des éléments de récepteur contenus dedans à travers le corps de support,
(e) immobiliser de façon spécifique par rapport au lieu et/ou au temps les éléments de récepteur dans des positions respectives prédéterminées sur le support de corps par illumination via une matrice de sources lumineuses programmable, et
(f) répéter les étapes (d) à (e) jusqu'à ce que les récepteurs souhaités aient été synthétisés dans les positions respectives prédéterminées.

2. Procédé selon la revendication 1, **caractérisé en ce que** le support de corps non marqué selon (a) est préparé par un fabricant et est livré à un client comme propriétaire de l'unité de synthèse selon (b).

3. Procédé selon l'une quelconque des revendications 1 à 2, **caractérisé en ce que** selon l'étape (c), les séquences sont extraites d'une base de donnés librement accessible.

4. Procédé selon l'une quelconque des revendications 1 ou 2, **caractérisé en ce que** selon l'étape (c), les séquences sont extraites d'une base de données préparée par le fabricant du corps de support non marqué selon (a).

5. Procédé selon l'une quelconque des revendications 1 à 4, **caractérisé en ce que** l'on utilise le support pour le dosage des analytes dans un échantillon.

6. Procédé selon la revendication 5, **caractérisé en ce que** plusieurs cycles de synthèse/dosage d'analyte sont réalisés, moyennant quoi les récepteurs d'un cycle suivant sont synthétisés sur la base des informations provenant d'un cycle précédent.

7. Procédé selon l'une quelconque des revendications 5 ou 6, **caractérisé en ce que** l'on dose un échantillon provenant d'un patient.

8. Procédé selon la revendication 7, **caractérisé en ce que** l'on équilibre les résultats du dosage dans les différences individuelles du patient et que l'on forme un second support à partir des résultats, lequel réalise un dosage adapté à chaque patient.

9. Procédé selon l'une quelconque des revendications précédentes, **caractérisé en ce que** l'on introduit le corps de support dans une unité de synthèse et d'analyse intégrée, **en ce que** l'on met le support en contact avec un échantillon contenant un analyte après la synthèse des récepteurs et **en ce que** l'on dose les analytes via leur liaison avec les récepteurs immobilisés sur le support.

10. Procédé selon l'une quelconque des revendications précédentes, **caractérisé en ce que** le corps de support contient au moins un canal.

11. Procédé selon l'une quelconque des revendications précédentes, **caractérisé en ce que** le corps de support contient une pluralité de canaux, qui sont disposés de préférence parallèlement les uns aux autres.

12. Procédé selon l'une quelconque des revendications précédentes, **caractérisé en ce que** les récepteurs sont choisis parmi des acides nucléiques et des analogues d'acide nucléique.

13. Procédé selon la revendication 12, **caractérisé en ce que** les éléments de récepteur sont choisis parmi des nucléotides, des oligonucléotides, des analogues de nucléotide et des analogues d'oligonucléotide.

14. Procédé selon l'une quelconque des revendications 1 à 11, **caractérisé en ce que** les récepteurs sont choisis parmi des polypeptides.

15. Procédé selon la revendication 14, **caractérisé en ce que** les éléments de récepteur sont choisis parmi des acides aminés et des peptides.

16. Procédé selon l'une quelconque des revendications précédentes, **caractérisé en ce qu'**un kit de réactifs, comprenant un support et des éléments pour la synthèse des récepteurs polymères, est mis en oeuvre sur le support.

17. Procédé selon l'une quelconque des revendications précédentes, **caractérisé en ce que** le corps de support est introduit dans une unité de synthèse, comprenant une matrice de sources lumineuses programmable, une matrice de détecteurs, un moyen pour l'apport du fluide dans le support et pour l'évacuation du fluide hors du support ainsi qu'un moyen de commande et de réglage électronique, moyennant quoi le corps de support est disposé entre les matrices de sources lumineuses et de détecteurs.

18. Procédé selon l'une quelconque des revendications précédentes, **caractérisé en ce que** l'unité de synthèse comprend en outre un moyen pour déprotéger les composants de réaction sur le support.

19. Utilisation du procédé selon l'une quelconque des revendications 1 à 18, pour le séquençage d'acides nucléiques.

20. Utilisation selon la revendication 19, pour le nouveau séquençage et/ou le reséquençage d'un matériau génétique complexe, comme un génome individuel ou des acides nucléiques synthétiques.

21. Utilisation du procédé selon l'une quelconque des revendications 1 à 18, pour l'obtention d'informations diagnostiques pour le traitement individuel d'un patient, comme l'action individuelle d'agents pharmaceutiques.

22. Utilisation du procédé selon l'une quelconque des revendications 1 à 18, pour l'analyse d'activité de substances pharmacologiques.

23. Utilisation du procédé selon l'une quelconque des revendications 1 à 18, pour la constitution et l'analyse de banques de substances.

24. Utilisation du procédé selon l'une quelconque des revendications 1 à 18, pour la comparaison d'individus dans une population.

25. Système de synthèse intégrée de récepteurs sur un support, comprenant :
(a) un corps de support,
(b) une unité de synthèse comprenant une matrice de sources lumineuses programmable,
(c) un moyen pour la synthèse de récepteurs grâce à une structure à étages des éléments de récepteur dans des positions respectives prédéterminées du support et
(d) une base de données pour extraire des informations sur les séquences des récepteurs à synthétiser sur le support.

26. Système selon la revendication 25, **caractérisé en ce que** le corps de support comprend au moins un canal.

27. Système selon l'une quelconque des revendications 25 ou 26, **caractérisé en ce que** l'unité de synthèse comprend en outre une matrice de détecteurs, un moyen pour l'apport de fluide dans le support et pour l'évacuation du fluide hors du support ainsi qu'un moyen de commande et de réglage électronique.

28. Système selon la revendication 27, **caractérisé en ce que** le corps de support est disposé entre la matrice de sources lumineuses programmable et la matrice de détecteurs.
